# EUROPEAN PATENT APPLICATION

(11) **EP 1 987 724 A1**
(43) Date of publication of application: **05.11.2008**
(21) Application number: 07707851.7
(22) Date of filing: 01.02.2007
(51) Int. Cl.: A23L 1/00, A23L 1/28, A23L 1/29, A61K 49/04

(54) **FOOD AND PROCESS FOR PRODUCING FOOD**

(30) Priority: 01.02.2006 JP 2006024332; 24.03.2006 JP 2006083367; 06.07.2006 JP 2006186855
(71) Applicant: Hiroshima Prefecture, Hiroshima-shi, Hiroshima 730-8511 (JP)
(72) Inventor: SAKAMOTO, Koji, Hiroshima-shi, Hiroshima, 731-0137 (JP); SHIBATA, Kenya, Hiroshima-shi, Hiroshima, 734-0014 (JP); ISHIHARA, Masako, Hiroshima-shi, Hiroshima, 734-0011 (JP); NAKATSU, Sayaka, Hiroshima-shi, Hiroshima, 732-0811 (JP)
(74) Representative: Minoja, Fabrizio
(86) International application number: PCT/JP2007/051665
(87) International publication number: WO 2007/088918

(57) **Abstract**

The present invention retains the original shape, color, taste, flavor and texture, suppresses liquating out nutritional contents, can easily adjust the hardness of a food to the desired hardness, does not require extreme care for deformation even though it is soft, is easy to handle during the manufacturing process, transportation and distribution process, can efficiently manufacture a processed food which uses a wide variety of food materials, can preserve by suppressing microbial deterioration due to its hygienic manufacture, particularly aims to promote appetite for the elderly and the like in oligotrophic conditions, and can laconically, easily and inexpensively manufacture a processed food in a short period of time, wherein the food is capable of supplying nutritious substance according to need. The method of manufacturing a food of the present invention including bringing either one or both of a thickener in a non-solvated state and microorganism which generates a viscous material into contact with the surface of a food material, and performing a pressure treatment, such that the food retains the shape of the food material and uniformly includes either one or both of a thickener in a non-solvated state and microorganism which generates a viscous material inside.

## Description

### Technical Field

The present invention relates to a food which retains the shape and texture of a food material, eases mastication and can suppress aspiration for those having difficulty with mastication and swallowing, and a process for producing a food, wherein the process suppresses the outflow of components included in such food material, discoloration and disappearance of flavor and can hygienically obtain a processed food. More specifically, the present invention relates to a food effective as an examination diet for medical use, wherein the diet can be used in examinations of mastication conditions, swallowing conditions, gastrointestinal tract activities, food movement speed and the like in those having difficulty with mastication and swallowing, and a process for producing thereof.

### Background Art

The elderly and weaning infants having difficulty with mastication and swallowing and the like generally consume a fluid diet, minced diet, jelly diet or food which is processed to be softened. These foods are processed and cooked to a state where one can chew with the gums, chew with the tongue, does not have to chew and the like. By the addition of a thickening agent and the like, it can be easily swallowed and the danger of aspiration is eliminated. For example, a process of obtaining a rice porridge for those having difficulty with mastication and swallowing, wherein the method coagulates or thickens rice water to encapsulate rice grain by using a coagulant and thickener, makes into rice porridge including rice grain and rice water, makes it into a gel whose overall hardness is set to the range of 5 x 10² N/m² to 5 x 10⁴ N/m² and makes it difficult for rice water and rice grain to separate in comparison with ordinary rice porridges (Patent Document 1), a method of processing preparations of Chinese medicine and herbal medicine for pharmaceutical use or for health food into any form of powders, granules, tablets, lumps and capsules by mixing at least either one of a thickener and gelator, wherein the medicine is prepared before using by dispersing the same in hot water or water (Patent Document 2), a method of using a swallowing aid which includes the range of 4 to 10 wt% heat-moisture treated starch and water, and is made into a uniform viscous liquid, sol or gel by performing a retort sterilization treatment (Patent Document 3), and a gel composition having properties suitable for swallowing, wherein the composition is prepared by mixing a solution of gelator, thickener and a solution of salts according to need, which are separately pre-heated, and cooling the same (Patent Document 4) have been reported.

However, these foods are not necessarily foods which have the taste, flavor, texture and the like possessed by the original food materials and hold visual palatability. Moreover, it is difficult to provide a food appropriately adjusted to the softness, more specifically, hardness required by each consumer.

On the other hand, a food prepared by vacuum cooking is used as a food for the elderly at facilities for nursing and social welfare for the aged, extended nursing care facilities for the aged and the like, wherein the food is prepared by seasoning and cooking pretreated food material and a seasoning solution in a state which is close to vacuum. This is because food material is cooked while preserving the texture, flavor and taste thereof by performing low temperature cooking to the food material (58°C to 95°C) by applying the principle that the thermal conductivity is raised and the boiling temperature is lowered when the inside of a film reaches a state which is close to vacuum, and because the food is hygienic and excellent in storage stability. Moreover, it enables to cook while suppressing the generation of active oxygen because it cooks the food material inside a film which is in a vacuum state. For example, a vacuum cooking method which prevents infectious food poisoning such as O-157, characterized in that the method stores lamp meat, sea bream and spiny lobster in a vacuum package and heats the same for 25 minutes in a steam oven whose temperature is set at 75°C (Patent Document 5), and a method of manufacturing an abalone subjected to packaging converting, wherein the abalone subjected to packaging converting is prepared by pressured heating after vacuum packaging a shucked abalone prepared by washing and adding the range of 0.03 to 0.05 wt% texture modifier, wherein the texture modifier includes the range of 13 to 18% of at least one kind of enzyme selected from the group consisting of papain, serratiopeptidase, streptokinase, α chymotrypsin, biotamylase and trypsin, the range of 43 to 63 wt% of common salt and the range of 22 to 42 wt% of starch (Patent Document 6) have been reported. However, by using these vacuum cooking methods, it is difficult to obtain a food which is sufficiently softened as a food for the elderly. Moreover, adjusting the hardness of food material to the desired softness and making adjustments in which nutritional contents such as calories and minerals are added in a single process has not reported yet.

The present inventors have already developed a method of introducing an enzyme into a tissue of a plant food material while immersing the plant food material in an enzyme solution under a reduced pressure to preserve its original form after freezing and thawing the same (Patent Document 7), a method of introducing an enzyme into a tissue by immersing a vegetable food in an enzyme solution under a reduced pressure, seasoning and sterilizing with heat and pressure (Patent Document 8) and the like. The food obtained by this method enables those having difficulty in mastication such as the elderly to relish hard food materials which are difficult to eat in a form where the food materials maintain their original shape, color, taste, flavor, texture and nutritional contents, can adjust its hardness of the food according to the degree of hardness required by consumers, and can be efficiently manufactured.

However, when a thawed food material is subjected to a reduced pressure treatment in an enzyme solution, there are cases where nutritional contents elute off as a drip. Moreover, because soft food materials whose hardness is adjusted are prone to deformation, extreme caution is required during the subsequent manufacturing process, packaging process and distribution process, and thus there is a problem in terms of handling. Furthermore, an enzyme and reaction conditions suitable for each food material are set when a reduced pressure treatment is performed. Therefore, in order to manufacture dishes which use a wide variety of food materials such as chop suey, sweet-sour pork and food boiled in a soy broth, there is an inefficient aspect where one performs a softening treatment for each food material and, subsequently, mixes the food materials in the cooking and packaging processes.

Moreover, even in a state where a vegetable food obtained by the method is cooked with the above described thickener which adds thickness, the thickener rarely penetrate the inside of the vegetable food itself, and the liquid included in the vegetable food itself remains in a state of low viscosity. Therefore, in those having difficulty in swallowing who consume the same, the liquid included in the vegetable food may be separated and generated in mouth by mastication, and there is a risk of aspiration. For those having difficulty in swallowing, there is a high level of demand for a safe food material which maintains its original texture without the risk of aspiration.

Furthermore, although the method can uniformly include a degradative enzyme inside the food material, the range of penetration thereof is restricted to the intercellular space and cell surface, and it is difficult to allow it to reach the inside of the cell. Therefore, it is difficult to degrade substrates inside the cell by the above described method. It is required to further develop a technology which allows a degradative enzyme to penetrate the inside of the cell in order to retain the shape of a food, further soften the food, and to ease consumption for those having difficulty with mastication and swallowing.

Conventionally, dielectric heating has been used for microwave ovens not only at food factories but also at common households for thawing, heating, drying and swelling treatment of food. A method of penetrating a seasoning solution by heating for a long period of time in a microwave oven (Patent Document 9) and a method of penetrating a seasoning by combining microwave heating and vacuum cooking (Patent Document 10) are disclosed and used as food cooking technologies. However, when a degradative enzyme is used in these methods, the degradative enzyme is denatured by heat and deactivated by microwave heating, and it is impossible to introduce the degradative enzyme without being denatured into the food.

Moreover, for those having difficulty in swallowing and the like, examinations of mastication conditions, swallowing conditions, gastrointestinal tract activities, food movement speed and the like are generally performed by making a subject to consume a food including a contrast agent and taking images by using imaging devices such as X-ray photography, CT, MRI and PET. As a method for a subject to consume a food including a contrast agent, a method of using a high density material consisting of hydrocarbons and proteins as a contrast agent and administering the same orally directly (Patent Document 11), a method of consuming a jelly and fluid diet wherein a contrast agent is mixed, or steamed bread coated with a contrast agent on the surface, method of consuming a food which is formed and processed by mixing a contrast agent in the processing step of a formed processed food such as biscuit (Patent Documents 12 and 13) and the like are used.

However, when the above described high density material consisting of hydrocarbons and proteins is used as a contrast agent, it detects conditions which are dissociated from the digestive conditions of digestive organs when they digest actual food materials. Moreover, the jelly wherein a contrast agent is mixed, steamed bread coated with a contrast agent on the surface, and biscuit wherein a contrast agent is mixed in the processing step, and the like cannot detect mastication conditions and swallowing conditions when a subject actually consumes food materials.

Any food material used in these methods is not in a state where it has the original shape of the food or food material. As long as one uses an administration method of a contrast agent using such food material, it cannot be said that imaging examination is conducted under the conditions where a subject actually consumes foods, for example, the conditions which accurately reflect the physical conditions, texture, flavor, taste, color and nutritional contents of the foods. Therefore, it is difficult to accurately examine the conditions under which a subject actually consumes foods, more specifically, mastication conditions, swallowing conditions, gastrointestinal tract activities and the like.
[Patent Document 1] Japanese Patent Laid-Open No. 11-187832
[Patent Document 2] Japanese Patent Laid-Open No. 11-322624
[Patent Document 3] Japanese Patent Laid-Open No. 2001-238651
[Patent Document 4] Japanese Patent Laid-Open No. 2002-300854
[Patent Document 5] Japanese Patent Laid-Open No. 2005-304451
[Patent Document 6] Japanese Patent Laid-Open No. 10-276729
[Patent Document 7] Japanese Patent No. 3686912
[Patent Document 8] Japanese Patent Laid-Open No.2006-223122
[Patent Document 9] Japanese Patent No. 3615747
[Patent Document 10] Japanese Patent Laid-Open No.2001-238612
[Patent Document 11] Japanese Patent Laid-Open No.6-228013
[Patent Document 12] Japanese Patent Laid-Open No.2002-71669
[Patent Document 13] Japanese Patent Laid-Open No.2003-284522

It is an object of the present invention to provide a process for producing a food, wherein the process allows the food to retain its original shape, color, taste, flavor and texture, suppresses liquating out nutritional contents, can easily adjust the hardness of the food to the desired hardness, does not require extreme care for deformation even though it is soft, is easy to handle during the producing process, transportation and distribution process, can efficiently produce a food which uses a wide variety of food materials, can preserve by suppressing microbial deterioration due to its hygienic manufacture, particularly aims to promote appetite for the elderly and the like in oligotrophic conditions, and can laconically, easily and inexpensively manufacture a processed food in a short period of time, wherein the processed food is capable of supplying nutritious substance according to need.

It is an object of the present invention to provide a food and a process for producing a food thereof, wherein the food retains its original shape, color, taste, flavor, texture and nutritional contents, uniformly raises the viscosity of not only the liquid included on the surface and near the surface but also the liquid included inside, aims to reduce the amount of the liquid separated by mastication or can eliminate separation of the liquid. Moreover, it is an object of the present invention to provide a process for producing a food, wherein the process can laconically, easily and inexpensively prepare a food in a short period of time, wherein the food is capable of suppressing aspiration in those having difficulty in mastication and swallowing.

Furthermore, it is an object of the present invention to provide a process for producing a food, wherein the process allows the food to retain the original shape, color, flavor, texture, nutritional contents and the like of the food material, to uniformly include a degradative enzyme not only near the surface of the food material but also uniformly between the tissues inside, and further even in the inside of the cell, and can efficiently obtain a soft food.

Moreover, it is an object of the present invention to provide an examination diet for medical use, wherein the diet can accurately and easily examine mastication conditions, swallowing conditions, gastrointestinal tract activities, food movement speed and the like of actual food materials in subjects having difficulty in mastication and swallowing, and a process for producing such an examination diet for medical use, wherein the process can laconically prepare such examination diet for medical use in a short period of time.

### Disclosure of the Invention

The present inventors conducted earnest research, focusing on performing a pressure treatment by using a reduced pressure when performing vacuum packaging of food and the like to uniformly include a degradative enzyme in a food material in order to retain the original shape, color, taste, flavor and texture, aim to promote appetite and enable to adjust the hardness of the food to the desired hardness so that it can be used as a food material which can suppress aspiration in those having difficulty in mastication and swallowing. As a result, the present inventors obtained findings that by putting a food material after freezing or after freezing-thawing and a degradative enzyme in a package material or putting a food material, wherein a degradative enzyme solution is adhered to the surface thereof, in a packaging material and performing vacuum packaging, the degradative enzyme can be uniformly introduced into the inside of the food material, deformation of the soft food material can be suppressed during the subsequent manufacturing process and distribution process, and the handling thereof can be extremely eased.

Moreover, the present inventors found that, when doing this, by adding a seasoning, thickener, nutritious substance and the like in the packaging material, these can be introduced at the same time as the degradative enzyme, a vacuum treatment can be performed simultaneously for a wide variety of food materials, and packaging can also be performed. Subsequently, the present inventors obtained findings that a processed food which retains the original taste, flavor and texture of the food material can be efficiently and hygienically manufactured by enabling cooking at low temperatures and suppressing liquating out, namely drip by vacuum heating of the inside of the packaging material in cooking after performing softening of the food material to the desired degree by the action of the degradative enzyme.

Moreover, the present inventors conducted research on uniformly introducing a thickener being used to add thickness to a food material and a viscous material generated by fermentation of a microorganism into the inside of the food material in order to suppress aspiration in those having difficulty in swallowing. They attempted to uniformly introduce thickness components into the inside of the tissues of the food material by performing a pressure treatment by a reduced pressure or applied pressure and the like after immersing a food material in water including the thickness components such as starch in a gel state and a viscous material generated by a microorganism, and allowing the same to adhere to the surface by applying and the like. However, even by performing a pressure treatment, it was difficult to obtain a food, wherein a thickener in a gel state and a viscous material generated by a microorganism are included inside the food tissues.

The present inventors conducted earnest research to find that a thickener and microorganism can be uniformly included inside the food material by using a thickener in a non-solvated state and a microorganism which generates a viscous material and adhering the same to the surface of the food material, or immersing in water including a thickener in a non-solvated state and a microorganism which generate a viscous material, and then performing a pressure treatment by a reduced pressure or applied pressure. Subsequently, the present inventors obtained findings that the original shape, color, taste, flavor, texture and nutritional contents can be retained and the liquid included inside is not separated by mastication or the amount of the liquid separated can be reduced. Therefore, the present inventors found that aspiration can be suppressed in those having difficulty in swallowing when they masticate the food. In particular, the present inventors obtained findings that the food including the degradative enzyme as well as the solvated thickener or the viscous material generated by the microorganism can suppress the risk of aspiration and asphyxiation, and QOL (quality of life) can be improved in those having difficulty in mastication such as the elderly.

Furthermore, the present inventors conducted earnest research on a method of manufacturing a food, wherein the method allows the food to retain its shape and texture and further soften the food material and wherein the food can be consumed by those having difficulty in mastication by a usual method. As a result, the present inventors found that liquid transfer and steam diffusion process are allowed to take place rapidly by performing dielectric heating of a food material ahead of the manufacturing process, which results in the evaporation of the liquid not only between the tissues of the food material but also inside the cell, the reduction of the liquid content and creation of a passage for an enzyme capable of penetrating the inside of the cell, and that a degradative enzyme can be uniformly and efficiently introduced not only between the tissues of the food material but also into the inside of the cell by the subsequent pressure treatment by an applied pressure or reduced pressure. The present inventors obtained findings that by performing dielectric heating of a food material, one can aim to reduce the processing time, dramatically increase the contact efficiency of an enzyme substrate existing at the center of the cell and a degradative enzyme, and can manufacture a soft food in a state where the shape of the food material is retained without causing color change of the food material and degradation such as the outflow of nutrients, which cannot be obtained when the liquid in the food material is evaporated by using common heat treatment methods for food material such as boiling, baking and steaming.

Furthermore, the present inventors found that it is further effective in improving the penetration efficiency of the enzyme to once cool the food material, whose temperature is raised by dielectric heating, to 60°C or less.

Moreover, the present inventors found that a contrast agent for medical use can be uniformly included inside the food material by performing a pressure treatment by a reduced pressure or applied pressure while adhering the contrast agent for medical use on the surface of the food material or immersing in a solution including the contrast agent for medical use with the above described degradative enzyme, thickener in a non-solvated state and the like. The present inventors obtained findings that mastication conditions, swallowing conditions, gastrointestinal tract activities, food movement speed and the like can be accurately and easily examined by using an examination food uniformly including a contrast agent for medical use not only on the surface or near thereof but also into the inside, and further in the inside of the cell. The present invention was achieved on the basis of these findings.

The process for producing a food of the present invention allows the food to retain the original shape, color, taste, flavor and texture, suppresses the liquating out nutritional contents, can easily adjust the hardness of the food to the desired hardness, does not require extreme caution for deformation even the food is soft, is easy to handle during the manufacturing process, transportation and distribution process, can efficiently manufacture a processed food which uses a wide variety of food materials, and can preserve by suppressing microbial deterioration due to its hygienic manufacture. In particular, the method aims to promote appetite for the elderly and the like in oligotrophic conditions, and can laconically, easily and inexpensively manufacture a processed food in a short period of time, wherein the food is capable of supplying nutritious substance according to need. Furthermore, the process can penetrate a degradative enzyme not only between the tissues of the food material but also into the inside of the cell, and can efficiently produce a further softened food.

The food of the present invention retains its original shape, color, taste, flavor, texture and nutritional contents, uniformly raise the viscosity of not only the liquid included on the surface and near the surface but also the liquid included inside, aims to reduce the amount of the liquid separated by mastication or can eliminate separation of the liquid. Moreover, the process for producing a food of the present invention can laconically, easily and inexpensively produce a food in a short period of time, wherein the food can suppress aspiration in those having difficulty in mastication and swallowing. Furthermore, the food is preferred as an examination diet for medical use, wherein the diet can accurately and easily examine mastication conditions, swallowing conditions, gastrointestinal tract activities, food movement speed and the like of actual food materials in those having difficulty in mastication and swallowing.

The present invention relates to a food which retains the shape of the food material, characterized in that the food uniformly includes either one or both of a thickener and microorganism which generates a viscous material inside. Moreover, the present invention preferably further uniformly includes a degradative enzyme inside, uniformly includes a contrast agent for medical use inside, and can be used as an examination diet for medical use.

Moreover, the present invention relates to a process for producing a food, characterized in that the process comprises bringing either one or both of a thickener in a non-solvated state and microorganism which generates a viscous material into contact with the surface of the food material; and performing a pressure treatment, such that the food retains the shape of the food material and uniformly includes either one or both of a thickener in a non-solvated state and microorganism which generates a viscous material inside.

Furthermore, the present invention relates to a process for producing a food, characterized in that the process comprises putting a food material brought into contact with a degradative enzyme and any one or more of a thickener in a non-solvated state, microorganism which generates a viscous material, nutritious substance and seasoning according to need, or putting a food material as well as a degradative enzyme, and any one or more of a thickener in a non-solvated state, microorganism which generates a viscous material, nutritious substance and seasoning according to need, in a packaging material for vacuum packaging such that the food retains the shape of the food material and includes the degradative enzyme inside uniformly; and cooking after softening the food material by the action of the degradative enzyme.

Furthermore, the process for producing a food of the present invention the food material is preferably used after dielectric heating, freezing, freezing-thawing or drying, or after dielectric heating followed by cooling to 60°C or less, then performing either one of freezing, freezing-thawing or drying.

The present invention relates to a process for producing a food, characterized in that the process comprises bringing a degradative enzyme into contact with the surface of the food material after dielectric heating, freezing, freezing-thawing or drying of the food material, or after dielectric heating followed by cooling to 60°C or less, then performing either one of freezing, freezing-thawing or drying the food material; and performing a pressure treatment, such that the food retains the shape of the food material and uniformly includes the degradative enzyme inside.

### Brief Description of the Drawings

Figure 1 is a diagram showing the break resistance in one example of the food of the present invention;
Figure 2 is a diagram showing the syneresis rate in one example of the food of the present invention;
Figure 3 is a diagram showing a light microscopic image of a tissue by light photomicroscopy in one example of the food of the present invention;
Figure 4 is a diagram showing a polarizing microscopic image of a tissue by polarizing photomicroscopy in one example of the food of the present invention;
Figure 5 is a diagram showing the break resistance for the food which is obtained in one example of the method of manufacturing a food of the present invention;
Figure 6 is a diagram showing the break resistance for the food which is obtained in one example of the method of manufacturing a food of the present invention;
Figure 7 is a diagram showing the break resistance for the food which is obtained in one example of the method of manufacturing a food of the present invention;
Figure 8 is a diagram showing the syneresis rate for the food which is obtained in one example of the method of manufacturing a food of the present invention;
Figure 9 is a diagram showing the aggregation rate for the food which is obtained in one example of the method of manufacturing a processed food of the present invention;
Figure 10 is a diagram showing the aggregation rate for the food which is obtained in one example of the method of manufacturing a processed food of the present invention;
Figure 11 is a diagram showing the physiologically active substance (the content of oligosaccharides in potato) in the food in Example 24 of the method of manufacturing a food of the present invention; and
Figure 12 is a diagram showing the physiologically active substance (the content of peptides in chicken white meat) in the food in Example 25 of the method of manufacturing a food of the present invention.

### Best Mode for Carrying Out the Invention

### [Food]

The food of the present invention is characterized in that the food retains the shape of the food material and uniformly includes either one or both of a thickener and microorganism which generates a viscous material inside.

Any food thickener having a hydrating action with the water included in a food material can be used as the thickener included in the food of the present invention. When a food material includes a degradative enzyme, viscosity may be reduced according to the type of the degradative enzyme included. Therefore, the thickener is preferred to arbitrarily select according to the type of the degradative enzyme included. More specifically, examples of the thickener include wheat starch, rice starch, corn starch, potato starch, tapioca starch, sweet potato starch, curdlan, gums, agar, gelatin and pectin. These can be used singularly or in combination of two kinds or more as long as they do not reciprocally inhibit the actions. More preferred is a thickener which exists in a dispersion state without being solvated in a solvent such as water and alcohol at room temperature.

The content of the above described thickener is preferably adjusted according to the amount of the liquid included in the food material, the type of degradative enzyme if used and the conditions of food consumers such as those having difficulty in swallowing. For example, it can be set to the range of 0.01 to 0.5 g per 100 g of food material.

As a microorganism which generates a viscous material to be included in the food of the present invention, any microorganism which generates a viscous material by fermentation can be used. More specifically, examples include lactic acid bacteria and Bacillus subtilis (bacillus natto). The content of these microorganisms in the food material can be arbitrarily selected according to the type of food material, the type of degradative enzyme if used and the conditions of food consumers such as those having difficulty in swallowing.

A food uniformly including a thickener or microorganism which generates a viscous substance inside as described above has an increased viscoelasticity and break resistance not only on the surface of the food but also across the entire food. Therefore, it promotes mastication when consumed and can be used, for example, as a food for prevention of obesity.

The food of the present invention preferably uniformly includes a degradative enzyme with the above described thickener and microorganism which generates a viscous material. By uniformly including the degradative enzyme in the food tissues of the food material, the degradation of the food tissues whose viscoelasticity and break resistance are raised by including the thickener can be eased. Furthermore, the degradative enzyme also has an effect of easing the introduction of the thickener into the food material and an effect of softening. Any degradative enzyme for proteins, hydrocarbons, lipids and the like can be used as the degradative enzyme. It is preferred to use by arbitrarily selecting according to the type of food material and the conditions of consumers. More specifically, enzymes having digestive and degradative action of food material, for example, enzymes which degrade proteins into amino acids and peptides such as proteases and peptidases, enzymes which degrade polysaccharides such as starch, cellulose, inulin, glucomannan, xylan, alginic acid, fucoidan and pectin into oligosaccharides such as amylase, glucanase, cellulase, pectinase, pectin esterase, hemicellulase, β-glucosidase, mannase, xylanase, alginate lyase, chitosanase, inulinase and chitinase, and enzymes which degrade lipids such as lipase are suitable. These can be used singularly or in combination of two kinds or more as long as they do not reciprocally inhibit the actions.

The content of the above described degradative enzyme is preferably adjusted depending on the required softness of the food according to the type of food material, the type of degradative enzyme and the conditions of food consumers such as those having difficulty in swallowing. For example, it can be set to the range of 0.001 to 0.5 g per 100 g of food material.

As the food material to be used in the present invention, any animal and plant food material can be used. It may be a raw food material or a food material which is heated and cooked such as being boiled, baked, steamed and fried. More specifically, food materials such as vegetables such as radishe, carrot, burdock, bamboo shoot, ginger, cabbage, Chinese cabbage, celerie, asparagus, welsh onion, onion, spinache, Brassica chinensis komatsuna, Japanese ginger, broccoli, cucumber, eggplant and marrow bean, tuber and roots such as potato, sweet potato and aroid, beans such as soy bean, red bean, fava bean and pea, grains such as rice and wheat, fruits such as orange, apple, peache, cherrie, pear, pineapple, banana and plum, mushrooms such as shiitake mushroom, shimeji mushroom, enoki mushroom, nameko mushroom and matsutake mushroom, fish and seafood such as red sea bream, tuna, horse mackerel, chub mackerel, sardine, squid, octopus, Manila clam and clam, meat such as chicken, pork and beef, and algae such as brown seaweed, kelp and laver can be exemplified. Furthermore, it may be a processed food thereof, which is prepared by processing the above described food materials and food materials. The processed food may be any of fish jelly product such as fish paste, pickle, daily dish, noodles, various confectioneries and the like.

The size of such food material is preferably 30 mm or less in each side if it is quasi-cubic and 30 mm or less in diameter if it is quasi-spherical in order to uniformly introduce the above described thickener, microorganism, degradative enzyme and the like into the center. However, it can be made to a size suitable for each food material. When it is a food material for industrial use, it may be processed to a size and form suitable for packaging and transportation. Moreover, it may be in a size suitable to for cooking and consumption.

The food of the present invention, wherein the food includes the above described thickener and the like, is preferably for those having difficulty in mastication and swallowing. The food of the present invention retains its original shape, color, taste, flavor, texture and nutritional contents. Not only the liquid included on the surface and near the surface and the liquid included inside are hydrated with the thickener and a viscous material generated by a microorganism uniformly included inside the food. The food can reduce or eliminate the amount of the liquid separated from the food and generated in mouth when consumers masticate the same. As used herein, the term "syneresis rate" refers to a proportion of the liquid weight separated from the food by mastication and generated in mouth without being solvated by the thickener and viscous material generated by the microorganism to the total weight of the food. The liquid weight can be defines as a measurement value of the weight of the liquid generated from the food when the food is pulverized by a homogenizing device.

The food of the present invention can be used as an examination diet for medical use by uniformly including a contrast agent for medical use inside.

A contrast agent for medical use wherein the contrast agent can be taken images by the irradiation of active energy rays by imaging devices for medical use such as X-ray photography, CT, MRI and PET is suitable for the above described contrast agent for medical use. More specifically, examples of the contrast agent for medical use include iopamidol, iohexol, ioversol, iomeprol, iopromide, ioxylan, iotroran, amidotrizoic acid, meglumine iotalamate, iotalamic acid, ioxaglic acid, meglumine ioxaglate, iodized poppy seed oil fatty acid ethyl ester, iopanoic acid, barium sulfate, meglumine gadopentetate, gadoteridol, ammonium iron citrate and ferumoxides.

The content of the contrast agent for medical use can be adjusted according to the type of food material, the swallowing conditions of subjects and the like. For example, it can be set to the range of 3 to 45 g per 100 g of food material.

The food of the present invention can be manufactured by the process for producing a food of the present invention described hereinafter.

### [Process for producing a food]

The process for producing a food of the present invention is
characterized in that the process comprises bringing either one or both of a thickener in a non-solvated state and microorganism which generates a viscous material into contact with the surface of a food material; and performing a pressure treatment, such that the food retains the shape of the food material and uniformly includes either one or both of the thickener in a non-solvated state and microorganism which generates a viscous material inside.

As a process of bringing either one or both of a thickener in a non-solvated state and microorganism which generates a viscous material into contact with the surface of a food material in the process for producing a food of the present invention, a dispersion solution including either one or both of a thickener in a non-solvated state and microorganism which generates a viscous material can be used. In preparation of the dispersion solution, it is prepared so that the thickener maintains its non-solvated state without allowing it to solvate. When the thickener is non-solvated, it can be easily introduced into the tissues of the food material, and can be uniformly introduced into the inside of the food material. Examples of dispersion solvent include water, alcohol and a solution, wherein the same are mixed. The concentration of the non-solvated thickener in the dispersion solution can be, for example, set to the range of 10 to 50 wt%, and it is preferably set to the range of 10 to 30 wt%.

The number of the microorganism included in the dispersion solution is not specifically limited. However, it is preferably approximately 10⁷ cells/ml because the greater the initial number of the microorganism, the earlier a viscous material is generated.

The process of preparing a dispersion solution can be a process, wherein the above described non-solvated thickener and microorganism which generates a viscous material are added to a dispersion solvent and arbitrarily mixed. When doing this, it can be cooled to, for example, the range of 4°C to 20°C in order to suppress the solvation of the thickener and fermentation of the microorganism.

The obtained dispersion solution is applied, immersed or sprayed to a food material. When a food material is used in a frozen state, a process such as immersion is preferred because it can simultaneously perform thawing. When a food material is used in a thawed state or without being frozen, a process such as application, spray and immersion can be used. The immersion time can be set, for example, to the range of 5 to 120 minutes, and the temperature thereof can be set, for example, to the range of 10 to 70°C.

When the thickener in a non-solvated state and microorganism which generates a viscous material are in a powder form, a process of adhering and spraying the thickener in a non-solvated state and microorganism which generates a viscous material to the surface of the food material can be used.

Furthermore, when the above described thickener and microorganism which generates a viscous material are brought into contact with a food material, it is preferred to also bring a degradative enzyme into contact with the food material. As a process of bringing the degradative enzyme into contact with the food material, a process of mixing it to a dispersion solution of the thickener and the like, and a process of separately preparing a dispersion solution of the degradative enzyme, and applying, spraying and immersing the same to the food material can be used. When the degradative enzyme is in a powder form, a process of sprinkling onto the food material and the like can be employed. When a dispersion solution of the degradative enzyme is prepared, the pH of the dispersion solution is preferably in the range of 4 to 10. To adjust the pH, pH adjusters such as organic acids and the salts thereof, and phosphate can be used. When the food material is immersed to an aqueous dispersion solution including the degradative enzyme, the immersion time can be set, for example, to the range of 1 to 120 minutes, and the temperature thereof can be set, for example, to the range of 4 to 80°C.

The amount of the degradative enzyme to be used can be adjusted according to the type of food material, the swallowing conditions of food consumers and the like. For example, when it is included in the above described dispersion solution, it can be set to the range of 0.01 to 5.0 wt% as the concentration of the degradative enzyme, and it is preferred to prepare an enzyme dispersion solution in the range of 0.1 to 2.5 wt% is prepared and to adjust by the amount of the enzyme dispersion solution to be used. When it is used as a powder form, it can be in the range of 0.001 to 0.5 g per 100 g of food material, for example.

Moreover, as nutritious substance to be used with the above described thickener and the like, nutrients which need to be supplied and the like can be arbitrarily selected and used according to the nutritional condition, condition of the disease, low calorie conditions and the like of consumers. More specifically, vitamins, minerals, high calorie substances such as emulsified oils, proteins, functional peptides such as peptides which enhance their taste by generating amino acids and peptides or lower elevated blood pressure, functional ingredients such as water-soluble dietary fiber and oligosaccharides, gluconate, sugar alcohol, cyclodextrins and the like, other nutritious supplements, and microorganisms and enzymes which generate these nutritious substances in some cases can be exemplified. The amount of the nutritious substance to be used can be determined according to the conditions of consumers. For example, in case of weaning infants, it is preferred to use a emulsified oil and adjust to the amount to be added so that approximately one third of the total amount of caloric intake can be supplied by oil and fat content.

In addition, a seasoning to be used with the above described thickener and the like is used for the seasoning which eases consumption. More specifically, salt, soy sauce, fermented soybean paste, sugars such as table sugar, amino acids, nucleic acids and the like, oil and fat, spices, coloring agents and the like can be exemplified.

These nutritious substances, seasoning and the like can be included in the above described dispersion solution including the non-solvated thickener and the like, sprinkled directly onto the food, or prepared separately as a dispersion solution including the same, and applied, sprayed, immersed and sprinkled onto the food material.

A food material is preferably used in a frozen state or a thawed state after freezing, and used after drying according to the type. By freezing the food material, the water included in the food material freezes and expands its volume in the tissues of the food material, pushes the tissues and, subsequently, pores are formed to ease the introduction of the thickener, degradative enzyme and the like into the tissues when it turns to the state of water. Therefore, it can efficiently perform the introduction of these materials into the food material in a short period of time.

As a process of freezing the food material, it is preferred to be performed at a freezing temperature at which ice crystals are generated inside the food material. For example, it can be performed at -5°C or less. If it is -5°C or less, either quick freezing or slow freezing can be applied. Taking into account to allow ice crystals to be uniformly distributed to the entire inside and not to deteriorate the texture, it is suitable to set it around - 15°C. Thirty minutes are sufficient for the freezing time if it can allow ice crystals to be uniformly distributed to the entire inside. However, it can be frozen for a longer period of time than this.

Moreover, with foods whose epidermis is thick such as beans, it is preferred to evaporate the water near the surface until the percentage of decrease in the water reaches approximately the range of 2 to 10 wt% after freezing because it can increase the introduction efficiency of the thickener and the like described below. Cold air drying, hot air drying and freeze drying are preferred to evaporate the surface water.

The above described frozen food can be used while remaining in a frozen state. However, it can be used after thawing. As a process of thawing a frozen food material, a process of leaving the food material to stand at room temperature may be performed. It can also be performed by heating in a constant temperature unit to shorten the thawing time. The thawing time can be shorted with a higher heating temperature. However, in order to retain its quality, it is preferred to be 60°C or less. Moreover, in case of freeze dried food material, pores formed by the evaporation of the liquid ease the penetration of the thickener and the like, and can dramatically increase the content of the thickener and the like. A hundred eighty minutes or less are sufficient for the freeze drying time, and approximately 60 minutes are preferred.

Furthermore, as a food material, ones subjected to a dielectric heating treatment in place of a freezing treatment such as freezing, freeze drying, drying and the like, or prior to these freezing treatments are preferred because they evaporate the liquid included between the tissues inside the food material and inside the cell and reduce the liquid content, and can penetrate the thickener and degradative enzyme and the like not only between the tissues of the food material but also into the inside the cell in a pressure treatment in the subsequent process. By performing dielectric heating of a food material, liquid transfer and steam diffusion process are allowed to take place rapidly, and the liquid not only between the tissues of the food material but also inside the cell are evaporated to reduce the liquid content. This creates a passage for an enzyme capable of penetrating the inside of the cell, and an enzyme can be uniformly and efficiently introduced not only between the tissues of the food material but also into the inside of the cell by the subsequent pressure treatment. By performing dielectric heating of a food material, one can aim to shorten the processing time, dramatically increase the contact efficiency of an enzyme substrate existing at the center of the cell and a degradative enzyme, and retain the shape of the food material without causing color change of the food material and degradation such as the outflow of nutrients, which cannot be obtained when the liquid in the food material is evaporated by using common heat treatment methods for food materials such as boiling, baking and steaming, and can uniformly and efficiently include the thickener, degradative enzyme and the like in the food material.

Either high frequency wave or microwave heating can be used as the above described dielectric heating. However, microwave dielectric heating is preferred, and it is preferred to use a microwave whose frequency is in the range of 300 MHz to 30 GHz (wavelength in the range of 1 cm to 1 m). The power output which performs dielectric heating can be arbitrarily adjusted in relation to the heating time. It can be adjusted by making the heating time long with a low power output and making the heating time short with a high power output.

As for the temperature during dielectric heating, the center temperature of the food material is preferably set to 60°C or more, and more preferably to the range of 80 to 100°C in order to obtain an evaporation effect of the liquid of the food material. It is necessary to set the heating time according to the food material, but heating for approximately 20 seconds is sufficient in case of short heating.

As a device performing such dielectric heating, a microwave oven being used at common households, oven for industrial use being used at stores, and microwave heater, reduced pressure microwave heater, applied pressure microwave heater and the like for mass production at factory level can be used.

It is preferred to perform dielectric heating so that it reduces the liquid content of the food material by 3 wt% or more to the food material. By reducing the liquid content of the food material by 3 wt% or more to the food material, liquid transfer and steam diffusion are sufficiently taken place in the food material and the displacement efficiency of the degradative enzyme and the air inside the food material described below can be dramatically increased. The reduction of the liquid content in the food material is preferably 60 wt% or less to the food material because it can suppress the shape and properties of the food material from being impaired. Moreover, dielectric heating is characterized in that it can dry foods, and drying takes place from the surface of the food material according to the degree of evaporation. With food materials whose exodermis is hard such as beans, dielectric heating may be effective because the displacement efficiency of the degradative enzyme and the air inside the food material is further increased. However, there are cases where excess dielectric heating impairs the shape and properties of the food material and severely impairs the quality thereof, and may adversely serve as a cause for reducing the displacement efficiency of the degradative enzyme and the air by a pressure treatment with any material. Therefore, excess drying needs to be avoided.

As used herein, a measurement value by a drying method by reduced pressure heating can be employed as the reduction of the liquid content in a food material.

It is preferred to cool a food material after dielectric heating because it allows the tissues and cells inside the food material expanded due to heating to contract and gives rise to expansion of the intercellular space of the food material and damage and loosening of the cells. The food material after dielectric heating is preferably cooled to 60°C or less so that enzyme deactivation does not take place when it is brought into contact with the degradative enzyme. When a pressure treatment in the subsequent process is not performed immediately after, it can be stored in the refrigerator.

The pressure treatment performed after bringing the thickener and the like into contact with a food material makes the thickener in a non-solvated state and microorganism which generates a viscous material to include inside the food material uniformly. The pressure treatment can be performed by using a reduced pressure or applied pressure, combining a reduced pressure and applied pressure, and repeating multiple times according to need. A reduced pressure is preferably approximately in the range of 10 to 60 mmHg in suction pressure, and an applied pressure is preferably in the range of 10 to 4000 atmospheric pressure. By a pressure treatment in the range, the thickener and the like can be uniformly introduced into the inside of the food material in a short period of time ranging from 5 to 60 minutes and the like. A pressure is preferably approximately at 700 atmospheric pressure because the solubilization of the thickener and sterilization can be performed without impairing the quality of the thickener, microorganism and degradative enzyme by heating to approximately 90°C in the subsequent process. Such pressure treatment suppresses the destruction of the tissues of the food material and can rapidly and uniformly introduce the thickener, microorganism, degradative enzyme and the like into the inside without performing heating of the food material.

The amount of the thickener, microorganism and degradative enzyme to be introduced into the inside of the food material by such pressure treatment is preferably around 1 g in total per 100 g of the food material.

The solvation of the thickener in a non-solvated state and fermentation of the microorganism are preferably performed after uniformly including the thickener in a non-solvated state and microorganism which generates a viscous material inside the food material by such pressure treatment. As the solvation of the thickener, example can include a method of heating in the range of 55°C to 125°C. By solvating the thickener introduced into the inside the food material, the liquid included in the food material binds to the thickener, and the amount of the liquid separated from the food when consumers masticate can be reduced or separation of the liquid can be suppressed. Moreover, fermentation of the microorganism can be performed by arbitrarily heating to the activation temperature of the microorganism. A viscous material generated by fermentation hydrates with the liquid included in the food material, and a similar effect to that of the thickener can be obtained.

In another embodiment of the process for producing a food of the present invention, it can be a process of performing a pressure treatment in a state where a food material is immersed to an aqueous dispersion solution wherein a thickener in a non-solvated state, a microorganism which generates a viscous material, a degradative enzyme and the like are dispersed, and uniformly including the thickener in a non-solvated state, the microorganism which generates a viscous material, the degradative enzyme and the like inside the food material.

The process is, so to speak, a process, wherein a process of bringing the degradative enzyme and the like to the food material and a pressure treatment process in the above described process for producing a food are conducted simultaneously, and can more efficiently manufacture a food.

Another embodiment of the process for producing a food of the present invention is characterized in that the process comprises putting a food material brought into contact with a degradative enzyme and any one or more of a thickener in a non-solvated state, microorganism which generates a viscous material, nutritious substance and seasoning according to need, or putting a food material as well as a degradative enzyme, and any one or more of a thickener in a non-solvated state, microorganism which generates a viscous material, nutritious substance and seasoning according to need, in a packaging material for vacuum packaging such that the food retains the shape of the food material and includes the degradative enzyme inside uniformly; and cooking after softening the food material by the action of the degradative enzyme.

As the degradative enzyme to be used in the process, the thickener in a non-solvated state, microorganism which generates a viscous material, nutritious substance and seasoning which are used according to need, ones similar to the ones used in the above described process for producing a food can be used. As the mode of use of the degradative enzyme and thickener in a non-solvated state and the like which are used according to need, the ones similar to the ones used in the above described process for producing a food can be exemplified. However, in comparison with the case where a pressure treatment is performed in a state while being immersed to an dispersion solution of the degradative enzyme and the like, the amount of the degradative enzyme to be used can be kept to the necessity minimum. The amount of degradative enzyme when it is used in a powder state is preferably adjusted depending on the hardness (softness) required for the food material according to the type of the food material, the type of the degradative enzyme and conditions of consumers. More specifically, example can include the range of 0.001 to 0.5 g per 100 g of food material.

Moreover, similar food materials with which the above described degradative enzyme and the like are brought into contact can be exemplified. Food material is preferably used after dielectric heating, freezing, freezing-thawing or drying, or after dielectric heating followed by cooling to 60°C or less, then performing either one of freezing, freezing-thawing or drying. The processes of dielectric heating, freezing, freezing-thawing, drying, or dielectric heating followed by cooling can be processes similar to the above described process for producing the food. Furthermore, a similar process can be exemplified as the process of bringing the food material into contact with the degradative enzyme and the like.

In the process for producing a food in the present invention, a packaging material which is used for vacuum packaging the above described food material and degradative enzyme is preferably capable of maintaining air tight and liquid tight. Examples include bags having flexibility (flexible packaging materials) and containers. As the material of these packaging materials, plastic, aluminum evaporation plastic and the like to improve air tightness can be exemplified.

To such packaging material, a food material is put as well as the degradative enzyme and any one or more of the thickener, microorganism which generates a viscous material, nutritious substance and seasoning according to need. Moreover, the food material with which the degradative enzyme and any one or more of the thickener, microorganism which generates a viscous material, nutritious substance and seasoning according to need is brought into contact is put in a packaging material. When doing this, in the packaging material, the thickener, microorganism which generates a viscous material, nutritious substance, seasoning and the like can be added furthermore. Subsequently, the packaging material is vacuum packaged. When a frozen food material is used to be put in a packaging material, it is preferred to be in a thawed or half-thawed state because it can uniformly include the degradative enzyme and the like in the food material. A process of vacuum packaging can be a process commonly used when vacuum packaging a food. More specifically, a process of using a common vacuum packaging machine, drawing the air inside the packaging material and sealing with a heat seal and the like can be exemplified. After the above described vacuum packaging, a pressure treatment by an applied pressure, reduced pressure and the like can be further performed.

Subsequently, the degradative enzyme introduced into the inside of a food material exerts its function to soften the tissues of the food material. When doing this, it can be at normal temperature. However, to activate its action, it can be arbitrarily heated. The heating temperature is preferably 60°C or less, and more preferably in the range of 40 to 50°C. Heating in the range can suppress the outflow of components from the food material such as a drip, discoloration and deterioration of flavor.

The subsequent cooking of the food material vacuum packaged in a packaging material can be performed by using a heating apparatus. By this cooking, cooking of the food material can be completely performed. However, it can be confined to the degree which deactivates the degradative enzyme, and wherein the food material is half-cooked. Deactivation of the degradative enzyme can be performed by heating to allow the temperature at the center of the food material to reach the minimum temperature at which the degradative enzyme is deactivated. Because it is heating in vacuum, the boiling temperature is lowered. Therefore, the heating temperature can be set to, for example, the range of 65 to 125°C. The cooking time of the food material is preferably the time which can deactivate the degradative enzyme at the center of the food material in relation to the heating temperature.

For a processed food which is half-cooked, sufficient heating such as microwave heating and heating in boiling water can be performed in a state of being vacuum packaged or in a state where the food is taken out from the packaging material just before consumers eat it.

When a non-solvated thickener and microorganism which generates a viscous material are used to the above described food material, the solvation of the thickener and fermentation of the microorganism are conducted by heating to activate the above described degradative enzyme, heating to deactivate the enzyme, heating to cook and the like, and the liquid inside the food material is made to be bound to the thickener and the like.

After the above described heat treatment, a processed food can be quick frozen, freeze dried and dried. By such treatment, the storage stability of the obtained processed food can be further improved.

Moreover, another embodiment of the process for producing a food of the present invention is characterized in that the process comprises bringing a degradative enzyme into contact with the surface of a food material after dielectric heating, freezing, freezing-thawing or drying, or after dielectric heating followed by cooling to 60°C or less, then performing either one of freezing, freezing-thawing or drying; and performing a pressure treatment, such that the food retains the shape of the food material and uniformly include the degradative enzyme inside.

The above mentioned ones, conditions and processes can be employed as the food material, degradative enzyme, thickener and microorganism and the like and dielectric heating, freezing, freezing-thawing, drying, dielectric heating followed by cooling to 60°C or less, contacting with the degradative enzyme and the like and a pressure treatment subjected to the food material to be used in the embodiment of the process.

The food obtained by the above described process for producing a food retains its original shape, color, taste, flavor and texture, suppresses the elution of nutritional contents, can have the hardness desired by a consumer, is easy to handle, can efficiently manufacture a processed food which uses a wide variety of food materials, and enables long term preservation by suppressing microbial deterioration. Furthermore, it aims to promote appetite for the elderly and the like in oligotrophic conditions, is capable of supplying nutritious substance according to need, suppresses aspiration in those having difficulty in mastication and swallowing and is suitable for weaning infants, patients with digestive diseases and the like.

Moreover, in the above described process for producing a food, by performing the contact of the food material and a non-solvated thickener, degradative enzyme and the like in the presence of a contrast agent for medical use, an examination diet for medical use can be produced. The contrast agent for medical use can be used as a solution, wherein it is dissolved or dispersed in water, alcohol or mixed solution, wherein it can be used by adding to the above described degradative enzyme dispersion solution or thickener dispersion solution.

The concentration of the contrast agent for medical use in the above described solution is preferably arbitrarily selected in order to adjust the amount of the solution of the contrast agent for medical use. For example, to make it around 10 g per 100 g of food material, it can be set to the range of 10 to 70 wt%, and preferably the range of 20 to 60 wt%. When the concentration of the contrast agent for medical use is in the range, appropriate contrast study can be performed with a small dosage. Moreover, the pH of the solution of the contrast agent for medical use is preferably in the range of 4.0 to 8.0.

### [Example 1]

Fifty g of bamboo shoot boiled in water was used. The bamboo shoot was frozen at -30°C. The bamboo shoot was then immersed to an aqueous dispersion water, wherein a 0.3 wt% enzyme (hemicellulase "Amano" 90: made by Amano Enzyme Inc.) heated to 50°C and a non-hydrated thickener at the range of 0 wt% to 30 wt% (raw potato starch) were dispersed, for 15 minutes. After thawing, in a state of being immersed, the pressure was reduced for 5 minutes (40 mmHg) by a vacuum pump, and an enzyme reaction was performed for 60 minutes. Subsequently, enzyme deactivation and hydration (gelatinization) of the thickener were performed by heating for 5 minutes at 100°C.

The hardness (the break resistance) and syneresis rate were measured for the obtained food.

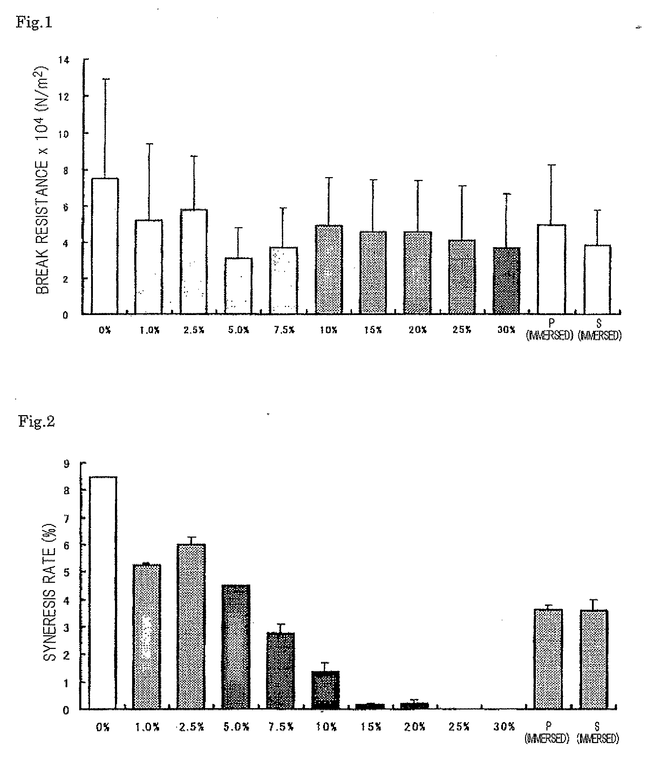

The break resistance was measured by Tensipresser (MODEL TTP-50 BXII: made by Taketomo Electric Inc.). The results are shown in Figure 1.

The syneresis rate was measured by measuring 20 g of the obtained bamboo shoot, homogenizing it with Stomacher (EXNIZER 400: made by Organo Co.) for 1 minute, leaving it to stand on a 100-mesh sieve for 5 minutes and measuring the amount of the liquid released, and evaluated as a proportion thereof to the total weight of the bamboo shoot (%). The results are shown in Figure 2.

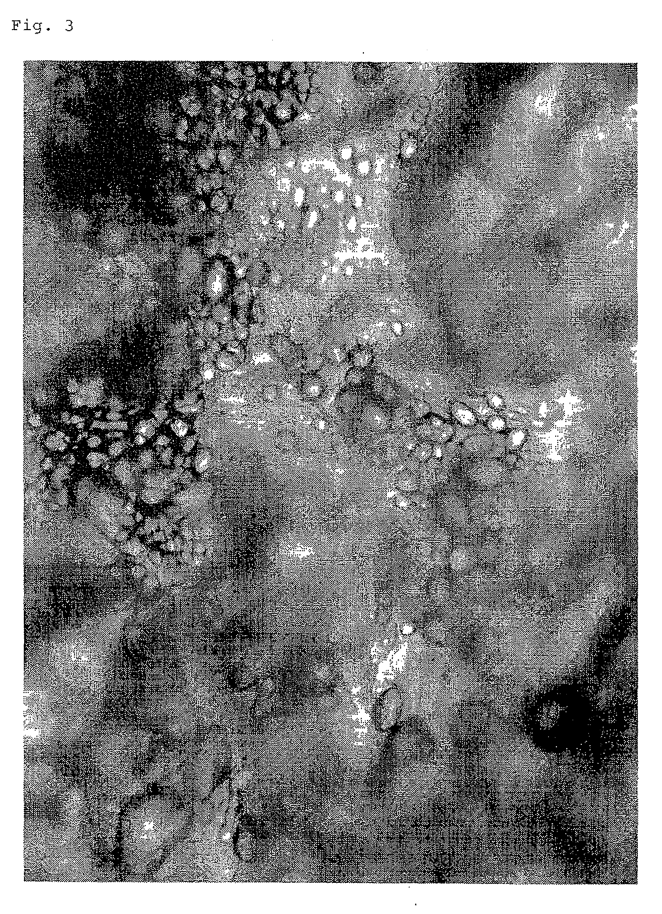

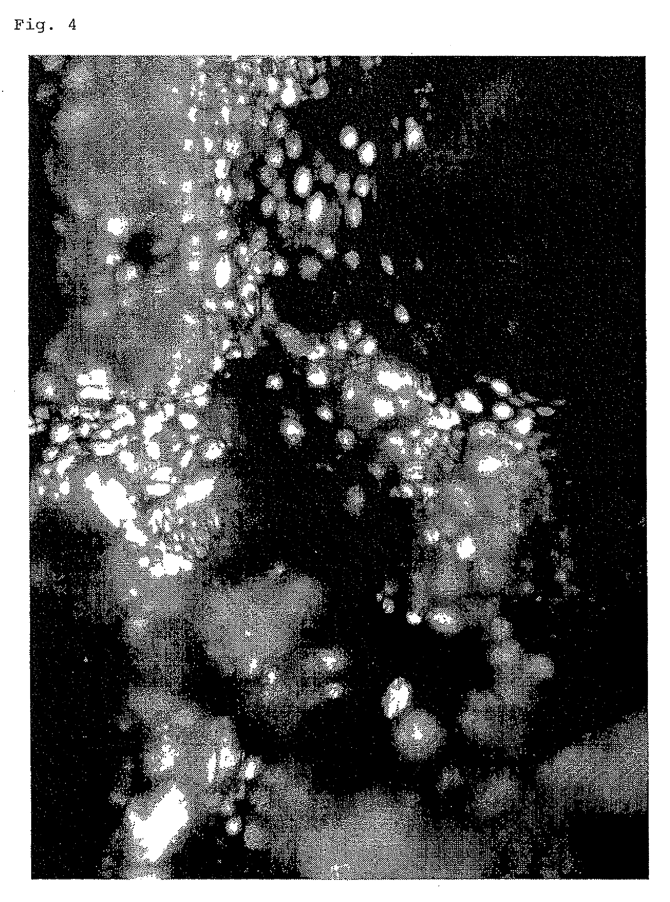

Furthermore, a light microscopic image and polarizing microscopic image were taken for the obtained food. Figure 3 shows a light microscopic image showing a state, wherein the thickener is introduced into the tissues of the bamboo shoot, taken by using a light microscope (OPTIPHOT-POL XTP-11: made by Nicon Corp.), and Figure 4 shows a microscopic image of the same position taken by using a polarizing microscope (OPTIPHOT-POL XTP-11: made by Nicon Corp.). It was shown that potato starch was uniformly included in the tissues of the bamboo shoot, particularly in the intercellular space leaving no space between.

### [Comparative Example 1-1]

A dispersion solution, wherein a bamboo shoot boiled in water was immersed prior to a pressure treatment, was used without adding a non-hydrated thickener (raw potato starch). After reduced pressure treatment, the bamboo shoot was immersed to an aqueous dispersion water of the 15 wt% non-hydrated thickener (raw potato starch) for 5 minutes. By performing the procedure similar to Example 1 except for above mentioned processes Food (P) was obtained. The break resistance and syneresis rate were measured for Food (P) by the procedure similar to Example 1. The results are shown in Figures 1 and 2.

### [Comparative Example 1-2]

Food (S) was obtained by performing a similar procedure to Comparative Example 1-2 except for using a cold water soluble type (Sunuswell α: made by Nihon Starch Co., Ltd) as the thickener. The break resistance and syneresis rate were measured for Food (S) by a method similar to Example 1. The results are shown in Figures 1 and 2.

These results clearly indicate the following. Determining the influence of the concentration of the non-solvated thickener (raw potato starch) in the dispersion solution on the break resistance of the bamboo shoot, the break resistance does not change as the concentration of the thickener increases (Figure 1). This indicates that the degradative enzyme is favorably introduced into the inside of the food material because viscosity increase does not take place even when the concentration of the thickener increases. Moreover, determining the influence of the concentration of the non-solvated thickener (raw potato starch) on the syneresis rate, the syneresis rate showed a tendency to be reduced as the concentration of the thickener increases, and the syneresis rate reached 0% when the thickener exceeded 25% (Figure 2). More specifically, the syneresis rate showed a tendency to be favorably reduced as the concentration of the non-solvated thickener increases. This indicates that even a relatively large particulate thickener is uniformly introduced into the inside of the food material because the degradative enzyme promotes intercellular loosening by performing a reduced pressure treatment simultaneously for the non-solvated thickener in a non-dissolved state and degradative enzyme. Therefore, it is clear that one can aim to reduce the syneresis rate while suppressing the increase in the break resistance.

### [Example 2]

A burdock was peeled, cut into 10 mm-thick rings, boiled in boiling water for 2 minutes, cooled in water and frozen at -15°C. Subsequently, it was immersed to a solution including 30 wt% raw wheat starch and a 0.5 wt% degradative enzyme (Cellulosin ME: made by HBI Enzymes Inc.) for 10 minutes at 40°C to thaw. While stirring (120 rpm), it was left to stand under a reduced pressure state (the initial pressure 40 mmHg) for 10 minutes at room temperature and returned to atmospheric pressure. After adding a seasoning solution (a commercially available broth consisting of soy sauce, extract of dried bonito shavings, table sugar, kelp extract and monosodium glutaminate) including a 1.5 wt% thickener (Sunuswell α: made by Nihon Starch Co., Ltd), it was packaged with deairation and sterilized with heat and pressure for 30 minutes at 95°C to manufacture a burdock for those having difficulty in swallowing.

The syneresis rate was measured for the obtained burdock by the procedure similar to Example 1. The syneresis rate was 0%, and it was in a state where it had a softened center. Therefore, it was suitable as a food for those having difficulty in swallowing.

### [Comparative Example 2]

A burdock was manufactured by performing the procedure similar to Example 2 except for using a solution including no raw wheat starch.

The syneresis rate was measured for the obtained burdock by the procedure similar to Example 1. The syneresis rate was 4%, and it was inappropriate as a food for those having difficulty in swallowing.

### [Example 3]

A commercially available bamboo shoot boiled in water was cut into quasi-triangle poles in 20 mm x 30 mm x 10 mm and frozen at -15°C. Subsequently, it was immersed to a solution including 20 wt% raw rice starch, a 0.3 wt% degradative enzyme (Pectinase G "Amano": made by Amano Enzyme Inc.) and a 4 wt% powder seasoning (a commercially available broth consisting of soy sauce, extract of dried bonito shavings, table sugar, kelp extract and monosodium glutaminate) for 15 minutes at 50°C to thaw. While agitating (120 rpm), it was left to stand under an applied pressure state (700 atmospheric pressure) for 10 minutes at room temperature and returned to atmospheric pressure. It was packaged with deairation without further processing and sterilized with heat and pressure for 30 minutes at 95°C to manufacture a bamboo shoot for those having difficulty in swallowing.

The syneresis rate was measured for the obtained bamboo shoot by the procedure similar to Example 1. The syneresis rate was 0%, and it was in a state where it had a softened center. Therefore, it was suitable as a food for those having difficulty in swallowing.

### [Comparative Example 3]

A bamboo shoot was produced by performing the procedure similar to Example 3 except for using a solution including no raw rice starch.

The syneresis rate was measured for the obtained bamboo shoot by the procedure similar to Example 1. The syneresis rate was 5%, and it was inappropriate as a food for those having difficulty in swallowing.

### [Example 4]

A commercially available raw beef was cut into 20 mm x 20 mm x 10 mm and frozen at -15°C. Subsequently, a solution including 20 wt% unheated gelatin and a 0.5 wt% protease (Papain W-40: made by Amano Enzyme Inc.) was sprayed to the surface of the raw beef to thaw. After being packaged, it was left to stand under a reduced pressure state (the initial pressure 40 mmHg) for 20 minutes at room temperature. After being left to stand for 30 minutes at 40°C, it was frozen to manufacture a beef.

Properties were measured after thawing the obtained beef. The beef showed properties suitable as a food for those having difficulty in swallowing in its hardness and ability to form alimentary bolus.

### [Comparative Example 4]

A beef was produced by performing the procedure similar to Example 4 except for using 20 wt% solvated gelatin instead of 20 wt% non-solvated gelatin.

The obtained beef was inferior in its ability to form alimentary bolus in comparison with the beef obtained in Example 4, and was inappropriate as a food for those having difficulty in swallowing.

### [Example 5]

Soy beans were heated for 30 minutes at 90°C without being cut, frozen for 16 hours at -15°C and subjected to freeze drying for 60 minutes. It was then immersed to a solution including Bacillus subtilis (bacillus natto) (10⁷ cells/ml) and a 1 wt% degradative enzyme (Macerozyme 2A: made by Yakult Pharmaceutical Industry Co., Ltd) for 10 minutes at 50°C to thaw. Subsequently, it was left to stand for 5 minutes at 50°C at an applied pressure state (1000 atmospheric pressure) and returned to atmospheric pressure. After fermentation for 20 hours at 37°C, soy beans for those having difficulty in swallowing were obtained.

The syneresis rate was measured for the obtained soy beans by a method similar to Example 1. The syneresis rate was 0%, and they were in a state where they had a softened center. Therefore, they were suitable as a food for those having difficulty in swallowing.

### [Comparative Example 5]

Soy beans were obtained by performing a similar procedure to Example 5 except for the fact that Bacillus subtilis (bacillus natto) (10⁷ cells/ml) was not used.

The obtained soy beans had a softened center, but were inferior in their ability to form alimentary bolus. Therefore, they were inappropriate as a food for those having difficulty in swallowing.

### [Example 6]

### [Preparation of a processed food]

A bamboo shoot boiled in water was cut into quasi-triangle poles in 20 mm x 1.5 mm x 10 mm and frozen at -30°C to manufacture a frozen bamboo shoot. A degradative enzyme solution was prepared by stirring (300 rpm) a degradative enzyme (hemicellulase "Amano" 90: made by Amano Enzyme Inc.) heated to 50°C in water so that the enzyme reached the range of 0.3 to 0.7 wt%. The frozen bamboo shoot was immersed to the degradative enzyme solution for 15 minutes to thaw. The thawed bamboo shoot was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation was performed by heating for 5 minutes at 98°C. The hardness (the break resistance) of the cooked bamboo shoot was measured by the following method.

### [The break resistance]

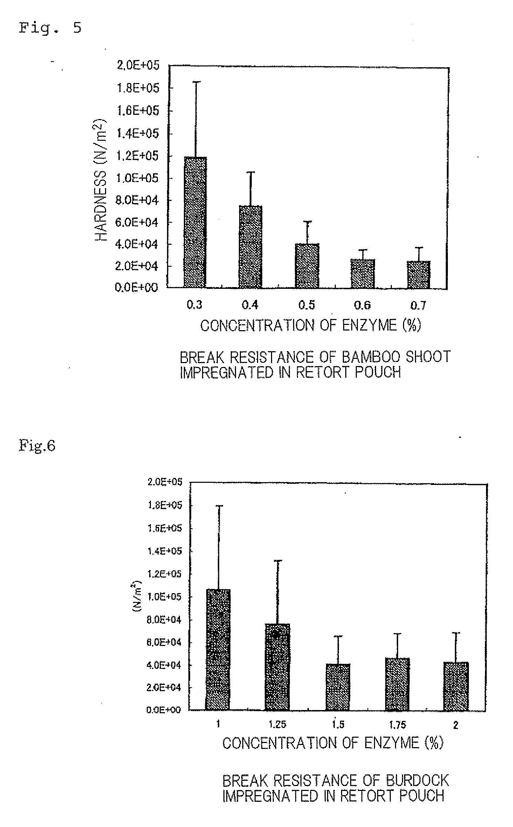

The break resistance was measured by Tensipresser (MODEL TTP-50 BXII: made by Taketomo Electric Inc.). The results are shown in Figure 5.

These results clearly indicate the following. Determining the influence of the concentration of the degradative enzyme solution being used for thawing by heat on the break resistance of the bamboo shoot, it shows a softening tendency as the concentration of the enzyme increases. When the concentration of the enzyme reaches 0.5 wt% or more, it is made possible for the hardness to satisfy the standard set by Ministry of Health, Labour and Welfare (5.0 x 10⁴ N/m² or less), wherein the standard permits to display that the food is for the elderly.

### [Example 7]

A burdock was peeled, cut into 5 mm-thick pieces on the angle, heated for 5 minutes by steam and frozen at -30°C to manufacture a frozen burdock. A degradative enzyme solution was prepared by stirring (300 rpm) a degradative enzyme (hemicellulase "Amano" 90: made by Amano Enzyme Inc.) heated to 50°C in water so that the enzyme reached the range of 1.0 to 2.0 wt%. The frozen burdock was immersed to the degradative enzyme solution for 15 minutes to thaw. The thawed burdock was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation was performed by heating for 5 minutes at 98°C.

The hardness (the break resistance) of the obtained burdock was measured by the procedure similar to Example 6. The results are shown in Figure 6.

These results clearly indicate the following. Determining the influence of the concentration of the degradative enzyme solution being used for thawing by heat on the break resistance of the burdock, it shows a softening tendency as the concentration of the enzyme increases. When the concentration of the enzyme reaches 1.5 wt% or more, it is made possible for the hardness to satisfy the standard set by Ministry of Health, Labour and Welfare (5.0 x 10⁴ N/m² or less), wherein the standard permits to display that the food is for the elderly.

### [Example 8]

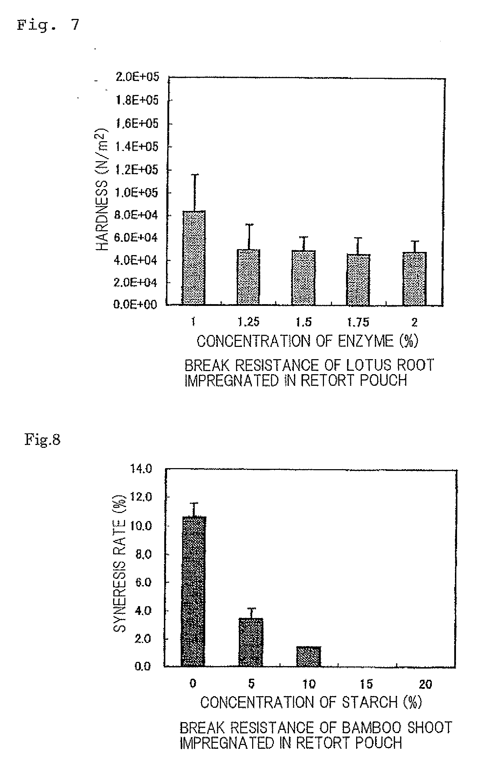

A lotus root boiled in water was cut into 20 mm x 1.5 mm x 10 mm and frozen at -15°C to manufacture a frozen lotus root. A degradative enzyme solution was prepared by stirring (300 rpm) a degradative enzyme (hemicellulase "Amano" 90: made by Amano Enzyme Inc.) heated to 50°C in water so that the enzyme reached the range of 1.0 to 2.0 wt%. The frozen lotus root was immersed to the degradative enzyme solution for 15 minutes to thaw. The thawed lotus root was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation was performed by heating for 5 minutes at 98°C. The hardness (the break resistance) of the obtained lotus root was measured by the procedure similar to Example 6. The results are shown in Figure 7.

These results clearly indicate the following. Determining the influence of the concentration of the degradative enzyme solution being used for thawing by heat on the break resistance of the lotus root, it shows a softening tendency as the concentration of the enzyme increases. When the concentration of the enzyme reaches 1.25 wt% or more, it is made possible for the hardness to satisfy the standard set by Ministry of Health, Labour and Welfare (5.0 x 10⁴ N/m² or less), wherein the standard permits to display that the food is for the elderly.

### [Example 9]

A bamboo shoot boiled in water was cut into quasi-triangle poles in 20
mm x 1.5 mm x 10 mm and frozen at -30°C to manufacture a frozen bamboo shoot. A degradative enzyme solution was prepared by stirring (300 rpm) a degradative enzyme (hemicellulase "Amano" 90: made by Amano Enzyme Inc.) heated to 50°C and a non-hydrated thickener (processed starch prior to gelatinization) in water so that the enzyme reached 0.3 wt% and the thickener reached the range of 0 to 20 wt%. The frozen bamboo shoot was immersed to the degradative enzyme solution for 15 minutes to thaw. The thawed bamboo shoot boiled in water was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation and hydration (gelatinization) of the thickener were performed by heating for 5 minutes at 95°C. The syneresis rate and aggregability of the obtained bamboo shoot was measured by the following method.

### [The syneresis rate]

The syneresis rate was measured by measuring 20 g of the obtained bamboo shoot, homogenizing it with Stomacher (EXNIZER 400: made by Organo Co.) for 1 minute, leaving it to stand on a 100-mesh sieve for 5 minutes and measuring the amount of the moisture released, and evaluated as a proportion thereof to the total weight of the bamboo shoot (%). The measurement results are shown in Figure 8.

These results clearly indicate the following. As the concentration of the non-hydrated thickener in the degradative enzyme solution being used for thawing by heat increases, the syneresis rate shows a tendency to be reduced. When the thickener reaches 15% or more, the syneresis rate reaches 0%.

### [Aggregability]

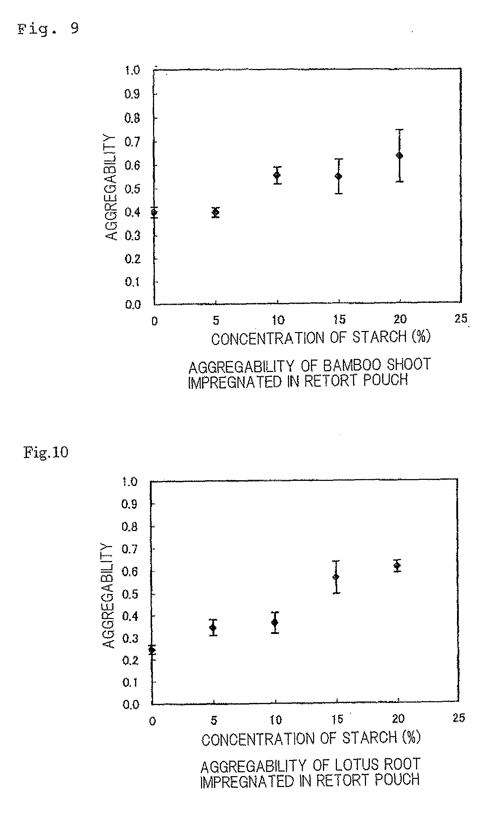

Aggregability, which serves as an indicator for cohesiveness of foods masticated in mouth, was measured by multiple integral byte analysis of a Tensipresser (MODEL TTP-50 BXII: made by Taketomo Electric Inc.). The measurement results are shown in Figure 9.

These results clearly indicate the following. As the concentration of the non-hydrated thickener in the degradative enzyme solution being used for thawing by heat increases, aggregability shows a tendency to be increased, and it can improve the ability to form alimentary bolus.

### [Example 10]

A lotus root boiled in water was cut into 20 mm x 1.5 mm x 10 mm and frozen at -15°C to manufacture a frozen lotus root. A degradative enzyme solution was prepared by stirring (300 rpm) a degradative enzyme (hemicellulase "Amano" 90: made by Amano Enzyme Inc.) heated to 50°C, a non-hydrated thickener (processed starch prior to gelatinization) in water so that the enzyme reached 1.0 wt% and the thickener reached the range of 0 to 2.0 wt%. The frozen lotus root was immersed to the degradative enzyme solution for 15 minutes to thaw. The thawed lotus root was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation and hydration (gelatinization) of the thickener were performed by heating for 5 minutes at 95°C. Aggregability of the obtained lotus root was measured by the procedure similar to Example 9. The results are shown in Figure 10.

These results clearly indicate the following. As the concentration of the non-hydrated thickener in the degradative enzyme solution being used for thawing by heat increases, aggregability shows a tendency to be increased, and it can improve the ability to form alimentary bolus.

### [Example 11]

A lotus root boiled in water was cut into 20 mm x 1.5 mm x 10 mm, frozen at -15°C and thawed to manufacture a thawed lotus root. In a flexible packaging material (150 x 250 mm), the thawed lotus root and 15 ml of a degradative enzyme solution including a 1.0 wt% degradative enzyme (hemicellulase "Amano" 90: made by Amano Enzyme Inc.) and a 5.0 wt% seasoning solution (a commercially available broth consisting of soy sauce, extract of dried bonito shavings, table sugar, kelp extract and monosodium glutaminate) were put, placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation and cooking were performed by heating for 15 minutes at 85°C.

The obtained cooked lotus root was stored in the refrigerator for 7 days at 4°C, heated for 10 minutes at 90°C, and the hardness (the break resistance) was measured by a method similar to Example 1. The obtained measurement value was 3.5 x 10⁴ N/m². This is a hardness favorable for mastication.

### [Example 12]

A burdock was peeled, cut into 8 mm-thick rings, heated for 5 minutes by steam and frozen at -30°C to manufacture a frozen burdock. A degradative enzyme solution was prepared by stirring (300 rpm) gradative enzyme (Cellulosin ME: made by HBI Enzymes Inc.) heated to 40°C, calcium and a seasoning (a commercially available broth consisting of soy sauce, extract of dried bonito shavings, table sugar, kelp extract and monosodium glutaminate) in water so that the enzyme reached 1.0 wt%, calcium reached 2.0 wt% and a seasoning reached 5.0 wt%. The frozen burdock was immersed to the degradative enzyme solution for 10 minutes to thaw. The thawed burdock was then taken out from the degradative enzyme solution, put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 45°C. Subsequently, enzyme deactivation and cooking were performed by heating for 10 minutes at 90°C.

The obtained cooked burdock was stored in the refrigerator for 3 days at 4°C, heated for 10 minutes at 90°C, and the hardness (the break resistance) was measured by the procedure similar to Example 6. The obtained measurement value was 3.0 x 10⁴ N/m². This is a hardness favorable for mastication.

It was confirmed that calcium is uniformly included in the inside of the cooked burdock as a result of analysis by an X-ray analysis microscope (XGT-5000: made by Horiba, Ltd.).

### [Example 13]

A chicken white meat was cut into 20 mm x 20 mm x 10 mm, heated, frozen at -20°C and thawed to manufacture a thawed chicken white meat. In a flexible packaging material (150 x 250 mm), the thawed chicken white meat and 13 ml of a degradative enzyme solution including a 0.5 wt% degradative enzyme (Bromelain F: made by Amano Enzyme Inc.), a 15 wt% liquid seasoning (a commercially available seasoning consisting of soy sauce, table sugar and kelp extract) and a 1.5% emulsified β carotene solution were put, placed in a vacuum state for 5 minutes by a vacuum packaging machine (V-307G-II: made by Tosei Electric Corporation), vacuum packaged and subjected to an enzyme reaction for 30 minutes at 40°C. Subsequently, enzyme deactivation and cooking were performed by heating for 10 minutes at 90°C.

The obtained cooked chicken white meat was stored in the freezer for 14 days at -30°C, thawed, heated for 5 minutes on a frying pan, and the hardness (the break resistance) was measured by the procedure similar to Example 6. In comparison with a chicken white meat to which a vacuum packaging process is not performed, it was softened and the hardness was approximately halved (2.5 x 10⁵ N/m²).

The content of β carotene in the cooked chicken white meat increased in comparison with a chicken white meat to which a vacuum packaging process is not performed as a result of measurement by the high performance liquid chromatography.

### [Example 14]

A raw beef round was cut into 20 mm x 20 mm x 10mm, frozen at - 20°C and thawed to manufacture a thawed raw beef round. 0.5 mass part of a degradative enzyme in powder (Protease N "Amano" G: made by Amano Enzyme Inc.) and 1.0 mass part of common salt was applied to 100 mass parts of the thawed raw beef round. It was then put in a flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 45°C. Subsequently, enzyme deactivation and cooking were performed by heating for 10 minutes at 90°C.

The obtained cooked beef was stored in the freezer for 7 days at - 30°C, thawed, heated for 5 minutes on a frying pan, and the hardness (the break resistance) was measured by the procedure similar to Example 6. It was softened in comparison with a cooked beef to which a vacuum packaging process is not performed, and the hardness (5.0 x 10⁵ N/m²) was approximately one third in comparison with that of the cooked beef. This is a hardness which is easy to chew.

### [Example 15]

A burdock was peeled, cut into 3 mm-thick pieces on the angle, heated for 5 minutes by steam and frozen at -30°C to produce a frozen burdock. A degradative enzyme solution was prepared by stirring a degradative enzyme (Cellulosin ME: made by HBI Enzymes Inc.) heated to 50°C, iron and a seasoning (a commercially available broth consisting of soy sauce, extract of dried bonito shavings, table sugar, kelp extract and monosodium glutaminate) in water so that the enzyme reached 2.0 wt%, iron reached 2.0 wt% and a seasoning reached 4.0 wt%. The frozen burdock was immersed to the degradative enzyme solution for 10 minutes to thaw. The thawed burdock was then taken out from the degradative enzyme solution, put in a flexible packaging material (150 x 250 mm), placed in a vacuum state for 3 minutes by a vacuum packaging machine (V-307G-II: made by Tosei Electric Corporation), vacuum packaged and subjected to an enzyme reaction for 60 minutes at 55°C. Subsequently, enzyme deactivation and cooking were performed by heating for 5 minutes at 90°C.

After the obtained cooked burdock was frozen for 48 hours at -30°C, the flexible packaging material was opened, and freeze drying was performed for 8 hours (FDU-830: made by Tokyo Rikakikai Co., Ltd.). After freeze drying, the cooked burdock was once again put in a flexible packaging material, vacuum packaged and preserved for 10 days at 10°C.

This was soaked in hot water by heating for 10 minutes in a hot water bath set at 100°C, and the hardness (the break resistance) was measured by the procedure similar to Example 6. As a result, the hardness was favorable for mastication.

It was confirmed that iron is uniformly included in the inside of the cooked burdock as a result of analysis by an X-ray analysis microscope (XGT-5000: made by Horiba, Ltd.).

### [Example 16]

Soy beans were heated for 30 minutes at 90°C without being cut, frozen at -20°C and subjected to freeze drying for 180 minutes. A degradative enzyme solution was prepared by stirring (300 rpm) a degradative enzyme (Macerozyme 2A: made by Yakult Pharmaceutical Industry Co., Ltd) heated to 40°C and Bacillus subtilis (bacillus natto) in water so that the enzyme reached 1.0 wt% and Bacillus subtilis reached (10⁷ cells/ml). The freeze dried soy beans were immersed to the degradative enzyme solution for 15 minutes. The freeze dried soy beans were then taken out from the degradative enzyme solution, put in a flexible packaging material (150 x 250 mm), placed in a vacuum state for 10 minutes by a vacuum packaging machine (V-307G-II: made by Tosei Electric Corporation), vacuum packaged and subjected to fermentation for 20 hours at 37°C.

The obtained food material was refrigerated for 3 days at 4°C, and the hardness (the break resistance) was measured by the procedure similar to Example 6. As a result, the hardness was favorable for mastication.

### [Example 17]

A bamboo shoot boiled in water was cut into quasi-triangle poles in 20 mm x 1.5 mm x 10 mm and frozen at -30°C to produce a frozen bamboo shoot. A degradative enzyme solution was prepared by stirring (300 rpm) a degradative enzyme (hemicellulase "Amano" 90: made by Amano Enzyme Inc.) heated to 50°C, a seasoning (a commercially available broth consisting of soy sauce, extract of dried bonito shavings, table sugar, kelp extract and monosodium glutaminate) and a non-hydrated thickener (processed starch prior to gelatinization) in water so that the enzyme reached 0.6 wt%, the seasoning reached 5.0 wt% and the thickener reached 20 wt%. The frozen bamboo shoot was immersed to the degradative enzyme solution for 15 minutes to thaw.

A burdock was peeled, cut into 8 mm-thick rings, heated for 5 minutes by steam and frozen at -30°C to produce a frozen burdock. A degradative enzyme solution was prepared by stirring (300 rpm) a degradative enzyme (Cellulosin ME: made by HBI Enzymes Inc.) heated to 50°C, a seasoning (a commercially available broth consisting of soy sauce, extract of dried bonito shavings, table sugar, kelp extract and monosodium glutaminate) and a non-hydrated thickener (processed starch prior to gelatinization) in water so that the enzyme reached 1.0 wt%, the seasoning reached 5.0 wt% and the thickener reached 15 wt%. The frozen burdock was immersed to the degradative enzyme solution for 15 minutes to thaw.

A carrot was cut into 8 mm-thick rings, heated for 5 minutes by steam and frozen at -30°C to manufacture a frozen carrot. A degradative enzyme solution was prepared by stirring (120 rpm) a degradative enzyme (Macerozyme 2A: made by Yakult Pharmaceutical Industry Co., Ltd) heated to 50°C, a seasoning (a commercially available broth consisting of soy sauce, extract of dried bonito shavings, table sugar, kelp extract and monosodium glutaminate) and a non-hydrated thickener (processed starch prior to gelatinization) in water so that the enzyme reached 0.2 wt%, the seasoning reached 5.0 wt% and the thickener reached 15 wt%. The frozen carrot was immersed to the degradative enzyme solution for 15 minutes to thaw.

The obtained thawed bamboo shoot, thawed burdock and thawed carrot were taken out from the degradative enzyme solution, put them in the same flexible packaging material (150 x 250 mm), placed in a vacuum state for 5 minutes by a vacuum packaging machine (V-307G-II: made by Tosei Electric Corporation), vacuum packaged and subjected to an enzyme reaction for 60 minutes at 50°C. Subsequently, enzyme deactivation, hydration of the thickener (gelatinization) and cooking were performed by heating for 10 minutes at 90°C.

The obtained processed food with a wide variety of food materials were stored in the freezer for 7 days at -30°C, thawed and cooked by heating for 10 minutes at 90°C. Subsequently, the hardness (the break resistance) and syneresis rate were measured by the procedure similar to Example 6.

The break resistance was 4.4 x 10⁴ N/m² in the bamboo shoot, 5.0 x 10⁴ N/m² in the burdock and 3.6 x 10⁴ N/m² in the carrot. The hardness of any of the foods was favorable for mastication. The syneresis rate of any of the foods could suppress syneresis. A seasoning was penetrated to the inside of any of the foods.

### [Example 18]

A cucumber was cut into 10 mm-thick rings and frozen at -15°C. It was then immersed to an aqueous solution including a 33 wt% contrast agent (lopamiron 300: made by Nihon Schering K.K.) and a 0.5 wt% degradative enzyme (Macerozyme 2A: made by Yakult Pharmaceutical Industry Co., Ltd) for 10 minutes at 50°C to thaw. Subsequently, it was immersed to an aqueous solution including a 33 wt% contrast agent (lopamiron 300: made by Nihon Schering K.K.), a 0.5 wt% degradative enzyme (Macerozyme 2A: made by Yakult Pharmaceutical Industry Co., Ltd) and a 1.5 wt% thickener (Sunuswell α: made by Nihon Starch Co., Ltd), left to stand under a reduced pressure state (the initial pressure 40 mmHg) for 5 minutes at room temperature and returned to atmospheric pressure. It was then mixed with 20 g of a solution including a 33 wt% contrast agent (Iopamiron 300: made by Nihon Schering K.K.) and 40 g of a seasoning solution (a commercially available broth consisting of soy sauce, extract of dried bonito shavings, table sugar, kelp extract and monosodium glutaminate), packaged with deairation and sterilized with heat and pressure for 30 minutes at 118°C to obtain an examination diet for medical use (Cucumber 1).

As a result of observation by an X-ray analysis microscope (XGT-5000: made by Horiba, Ltd.), the obtained cucumber uniformly included the contrast agent even at the center thereof.

When a subject having difficulty in swallowing consumed the obtained examination diet for medical use (Cucumber 1), it was able to favorably consume and swallow the same. As a result of performing a swallowing imaging examination (video fluorography: VF) by X-ray, a favorable swallowing contrast image was obtained.

### [Comparative Example 18-1]

A cucumber was cut into 10 mm-thick rings and frozen at -15°C. It was then immersed to an aqueous solution including a 33 wt% contrast agent (Iopamiron 300: made by Nihon Schering K.K.) and a 0.5 wt% degradative enzyme (Macerozyme 2A: made by Yakult Pharmaceutical Industry Co., Ltd) for 10 minutes at 50°C to thaw. Subsequently, it was immersed to an aqueous solution including a 33 wt% contrast agent (Iopamiron 300: made by Nihon Schering K.K.), a 0.5 wt% degradative enzyme (Macerozyme 2A: made by Yakult Pharmaceutical Industry Co., Ltd) and a 1.5 wt% thickener (Sunuswell α: made by Nihon Starch Co., Ltd) and left to stand for 5 minutes at 50°C. When the contrast agent in the obtained cucumber was detected by the procedure similar to Example 18, the contrast agent was not included at the center thereof. A contrast image was not obtained when a swallowing imaging examination was performed by the procedure similar to Example 18.

### [Example 19]

A burdock was peeled, cut into 10 mm-thick rings, boiled in boiling water for 2 minutes, cooled in water and frozen at -15°C. Subsequently, it was immersed to an aqueous solution including a 33 wt% contrast agent (Oypalomin 300: made by Fuji Pharma Co., Ltd.) for 10 minutes at 50°C to thaw. It was then left to stand under a reduced pressure state (the initial pressure 40 mmHg) for 5 minutes at room temperature and returned to atmospheric pressure. It was then mixed with 20 g of a solution including a 33 wt% contrast agent (Oypalomin 300: made by Fuji Pharma Co., Ltd.) and 40 g of a seasoning solution (a commercially available broth consisting of soy sauce, extract of dried bonito shavings, table sugar, kelp extract and monosodium glutaminate), packaged in a retort pouch with deairation and sterilized with heat and pressure for 30 minutes at 118°C to obtain an examination diet for medical use (Burdock 2).

As a result of observation by an X-ray analyzer (XGT-5000: made by Horiba, Ltd.), the obtained burdock uniformly included the contrast agent even at the center thereof.

When a swallowing imaging examination (video fluorography: VF) by X-ray was performed for a subject who consumed the obtained examination diet for medical use (Burdock 2), a favorable swallowing contrast image was obtained.

### [Comparative Example 19-1]

A burdock was peeled, cut into 10 mm-thick rings, boiled in boiling water for 2 minutes, cooled in water and frozen at -15°C. Subsequently, it was immersed to an aqueous solution including a 33 wt% contrast agent (Oypalomin 300: made by Fuji Pharma Co., Ltd.) for 15 minutes at 50°C to thaw. When the contrast agent in the obtained burdock was detected by the procedure similar to Example 19, the contrast agent was not included at the center thereof. A contrast image was not obtained when a swallowing imaging examination was performed by the procedure similar to Example 19.

### [Example 20]

A commercially available bamboo shoot boiled in water was cut into quasi-triangle poles in 20 mm x 30 mm x 10 mm and frozen at -15°C. Subsequently, it was immersed to an aqueous solution including a 33 wt% contrast agent (Iopamiron 300: made by Nihon Schering K.K.) and a 0.1 wt% degradative enzyme (hemicellulase "Amano" 90: made by Amano Enzyme Inc.) for 10 minutes at 50°C to thaw. Subsequently, it was immersed to an aqueous solution including a 33 wt% contrast agent (Iopamiron 300: made by Nihon Schering K.K.), a 0.1 wt% degradative enzyme (hemicellulase "Amano" 90: made by Amano Enzyme Inc.) and a 1.5 wt% thickener (Sunuswell α: made by Nihon Starch Co., Ltd), left to stand under a reduced pressure state (the initial pressure 40 mmHg) for 5 minutes at room temperature and returned to atmospheric pressure. It was preserved for 60 minutes in a constant temperature unit set at 50°C. It was then mixed with 20 g of a solution including a 33 wt% contrast agent (Iopamiron 300: made by Nihon Schering K.K.) and 40 g of a seasoning solution (a commercially available broth consisting of soy sauce, extract of dried bonito shavings, table sugar, kelp extract and monosodium glutaminate), packaged with deairation and sterilized with heat and pressure for 30 minutes at 118°C to obtain an examination diet for medical use (Bamboo shoot 3).

As a result of observation by an X-ray analysis microscope (XGT-5000: made by Horiba, Ltd.), the obtained bamboo shoot uniformly included the contrast agent even at the center thereof.

When a subject having difficulty in swallowing consumed the obtained examination diet for medical use (Bamboo shoot 3), it was able to favorably consume and swallow the same. As a result of performing a swallowing imaging examination (video fluorography: VF) by X-ray, a favorable swallowing contrast image was obtained.

### [Comparative Example 20-1]

A commercially available bamboo shoot boiled in water was cut into quasi-triangle poles in 20 mm x 30 mm x 10 mm and frozen at -15°C. Subsequently, it was immersed to an aqueous solution including a 33 wt% contrast agent (Iopamiron 300: made by Nihon Schering K.K.) and a 0.1 wt% degradative enzyme (hemicellulase "Amano" 90: made by Amano Enzyme Inc.) for 10 minutes at 50°C to thaw. Subsequently, it was immersed to an aqueous solution including a 33 wt% contrast agent (Iopamiron 300: made by Nihon Schering K.K.), a 0.1 wt% degradative enzyme (hemicellulase "Amano" 90: made by Amano Enzyme Inc.) and a 1.5 wt% thickener (Sunuswell α: made by Nihon Starch Co., Ltd), and left to stand for 5 minutes at 50°C. When the contrast agent in the obtained bamboo shoot was detected by the procedure similar to Example 20, the contrast agent was not included at the center thereof. A contrast image was not obtained when a swallowing imaging examination was performed by the procedure similar to Example 20.

### [Example 21]

A commercially available raw beef was cut into 20 mm x 20 mm x 10 mm and frozen at -15°C. Subsequently, it was immersed to an aqueous solution including a 33 wt% contrast agent (Iopamiron 300: made by Nihon Schering K.K.) and a 0.5 wt% degradative enzyme (Papain W-40: made by Amano Enzyme Inc.) for 10 minutes at 50°C to thaw. Subsequently, it was immersed to an aqueous solution including a 33 wt% contrast agent (Iopamiron 300: made by Nihon Schering K.K.), a 0.5 wt% degradative enzyme (Papain W-40: made by Amano Enzyme Inc.) and 1 wt% agar, left to stand under a reduced pressure state (the initial pressure 40 mmHg) for 5 minutes at room temperature and returned to atmospheric pressure. It was then mixed with 20 g of a solution including a 33 wt% contrast agent (Iopamiron 300: made by Nihon Schering K.K.) and 40 g of a seasoning solution (a commercially available broth consisting of soy sauce, extract of dried bonito shavings, table sugar, kelp extract and monosodium glutaminate), packaged with deairation and sterilized with heat and pressure for 30 minutes at 118°C to obtain an examination diet for medical use (Beef 4).

As a result of observation by an X-ray analysis microscope (XGT-5000: made by Horiba, Ltd.), the obtained beef uniformly included the contrast agent even at the center thereof.

When a subject having difficulty in swallowing consumed the obtained examination diet for medical use (Beef 4), it was able to favorably consume and swallow the same. As a result of performing a swallowing imaging examination (video fluorography: VF) by X-ray, a favorable swallowing contrast image was obtained.

### [Comparative Example 21-1]

A commercially available raw beef was cut into 20 mm x 20 mm x 10 mm and frozen at -15°C. Subsequently, it was immersed to an aqueous solution including a 33 wt% contrast agent (Iopamiron 300: made by Nihon Schering K.K.), a 0.5 wt% degradative enzyme (Papain W-40: made by Amano Enzyme Inc.) and 1 wt% agar for 15 minutes at 50°C to thaw. When the contrast agent in the obtained beef was detected by the procedure similar to Example 21, the contrast agent was not included at the center thereof. A contrast image was not obtained when a swallowing imaging examination was performed by the procedure similar to Example 4.

### [Example 22]

The edible part of a pineapple was cut into 20 mm x 30 mm x 10 mm, heated for 2 minutes by steam, cooled in water and frozen at -15°C. After it was thawed at room temperature, an aqueous solution including a 33 wt% contrast agent (Iopamiron 300: made by Nihon Schering K.K.) and 1 wt% agar was sprayed to the surface in a proportion of approximately 10 wt% to the food material, and packaged in a film with deairation. This was left to stand for 30 minutes at room temperature to obtain an examination diet for medical use (Pineapple 5).

As a result of observation by an X-ray analysis microscope (XGT-5000: made by Horiba, Ltd.), the obtained pineapple uniformly included the contrast agent even at the center thereof.

When a subject having difficulty in swallowing consumed the obtained examination diet for medical use (Pineapple 5), it was able to favorably consume and swallow the same. As a result of performing a swallowing imaging examination (video fluorography: VF) by X-ray, a favorable swallowing contrast image was obtained.

### [Comparative Example 22-1]

The edible part of a pineapple was cut into 20 mm x 20 mm x 20 mm, heated for 2 minutes by steam, cooled in water and frozen at -15°C. After it was thawed at room temperature, an aqueous solution including a 33 wt% contrast agent (Iopamiron 300: made by Nihon Schering K.K.) and 1 wt% agar was sprayed to the surface in a proportion of approximately 10 wt% to the food material, packaged in a film and left to stand for 30 minutes at room temperature. When the contrast agent in the obtained pineapple was detected by the procedure similar to Example 22, the contrast agent was not included at the center thereof. A contrast image was not obtained when a swallowing imaging examination was performed by the procedure similar to Example 22.

### [Example 23]

Marrow beans were heated for 30 minutes at 90°C without being cut, frozen for 16 hours at -15°C and subjected to freeze drying for 60 minutes. It was then immersed to an aqueous solution including a 33 wt% contrast agent (Iopamiron 300: made by Nihon Schering K.K.), a 1 wt% degradative enzyme (hemicellulase "Amano" 90: made by Amano Enzyme Inc.) and 1.5 wt% agar for 10 minutes at 50°C to thaw. Subsequently, it was immersed to an aqueous solution including a 33 wt% contrast agent (Iopamiron 300: made by Nihon Schering K.K.), a 1 wt% degradative enzyme (hemicellulase "Amano" 90: made by Amano Enzyme Inc.) and 1.5 wt% agar, left to stand under an applied pressure state (1000 atmospheric pressure) for 5 minutes at 50°C and returned to atmospheric pressure. It was packaged with deairation and sterilized with heat and pressure for 30 minutes at 118°C to obtain an examination diet for medical use (Marrow bean 6).

As a result of observation by an energy dispersive X-ray spectrometer, the obtained marrow beans uniformly included the contrast agent even at the center thereof.

When a subject having difficulty in swallowing consumed the obtained examination diet for medical use (Marrow beans 6), it was able to favorably consume and swallow the same. As a result of performing a swallowing imaging examination (video fluorography: VF) by X-ray, a favorable swallowing contrast image was obtained.

### [Comparative Example 23-1]

Marrow beans were heated for 30 minutes at 90°C without being cut, frozen for 16 hours at -15°C and subjected to freeze drying for 60 minutes. It was then immersed to an aqueous solution including a 33 wt% contrast agent (Iopamiron 300: made by Nihon Schering K.K.), a 1 wt% degradative enzyme (hemicellulase "Amano" 90: made by Amano Enzyme Inc.) and 1.5 wt% agar for 15 minutes at 50°C to thaw. When the contrast agent in the obtained marrow beans was detected by the procedure similar to Example 23, the contrast agent was not included at the center thereof. A contrast image was not obtained when a swallowing imaging examination was performed by the procedure similar to Example 23.

### [Example 24]

A potato was cut into quasi-cylinders whose diameter was 2 cm and height was 1.5 cm. A total weight of 25.5 g was heated in a microwave oven (NE-SV30HA: made by Matsushita Electric Industrial Co., Ltd.) at 700 W for 60 seconds. After it was cooled so that the product temperature reached 30°C, the water content of the potato was measured 70.4 wt%. It was measured 79.7 wt% before dielectric heating. After the potato was immersed to an enzyme solution (liquefying enzyme 6T: made by HBI Enzymes Inc.) having amylase activity prepared at 0.5 wt% by using a citrate buffer solution (pH 5.0) for 5 minutes, it was put in a pressure resistance container while immersing the potato in the enzyme solution and subjected to a reduced pressure treatment (60 mmHg) by a vacuum pump for 5 minutes. The potato was returned to atmospheric pressure, taken out from the enzyme solution and subjected to a reaction for 1 hour in a constant temperature bath set at 70°C. After the reaction, enzyme deactivation was performed by heating for 5 minutes at 100°C. The obtained potato maintained the shape prior to the treatment.

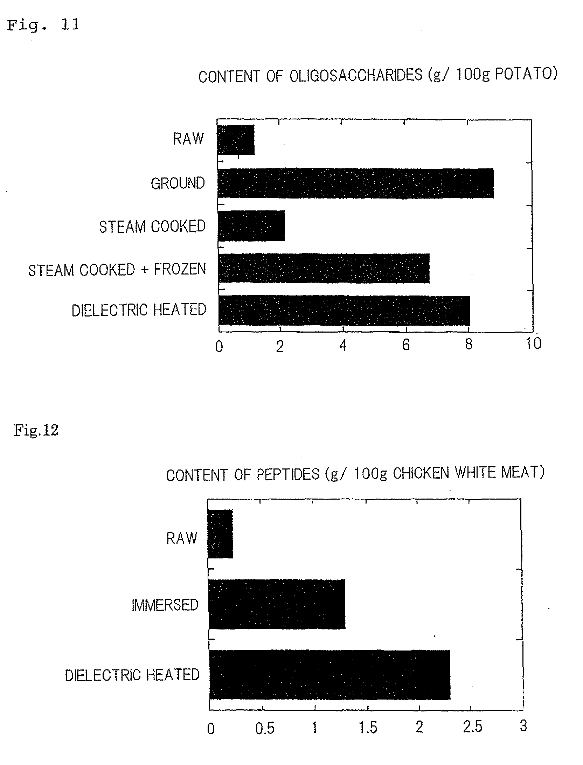

The manufactured potato was ground and subjected to aqueous extraction. The content of oligosaccharides included in the potato was measured by high performance liquid chromatography (SHODEX SUGAR KS-802 column: made by Showa Denko K.K.). The content of oligosaccharides was 8.04 g in 100 g of the potato. The content of oligosaccharides was measured by calculating the total amount of disaccharides through decasaccharides excluding monosaccharides by glucose equivalent. The results are shown in Figure 11 (dielectric heated).

### [Comparative Example 24-1]

25.1 g of an untreated potato was ground and subjected to aqueous extraction without further processing, and the content of oligosaccharides in the raw potato was measured by the procedure similar to Example 24. The content of oligosaccharides was 1.21 g in 100 g of the potato. The results are shown in Figure 11 (raw).

### [Comparative Example 24-2]

23.3 g of a potato was cut into similar cylinders, heated for 5 minutes by steam, cooled so that the product temperature reached 30°C and ground. The ground product was mixed with an enzyme solution prepared by a similar method (mixed in a mass ratio of potato: enzyme solution = 3: 1) and subjected to a reaction for 5 minutes at 70°C. Immediately after the reaction, enzyme deactivation was performed by heating at 100°C, and the content of oligosaccharides was measured by the procedure similar to Example 24. The content of oligosaccharides was 8.80 g in 100 g of the potato. The results are shown in Figure 11 (ground). In addition, the 5-minute reaction time is a condition which generates the largest amount of oligosaccharides under the same condition. After confirming that excess degradation took place therefore the content of oligosaccharides decreases meanwhile the content of monosaccharides increases when the enzyme reaction time was longer, it was used as a comparative control.

### [Comparative Example 24-3]

23.3g of a potato was cut into similar cylinders, heated for 5 minutes by steam, and cooled so that the product temperature reached 30°C. Subsequently, it was immersed to an enzyme solution by the procedure similar to Example 24, subjected to a reduced pressure treatment, enzyme reaction and enzyme deactivation. The content of oligosaccharides was measured from the obtained potato by the procedure similar to Example 24. The content of oligosaccharides was 2.14 g in 100 g of the potato. The results are shown in Figure 11 (steam cooked).

### [Comparative Example 24-4]

22.9g of a potato was cut into similar cylinders, heated for 5 minutes by steam, cooled so that the product temperature reached 30°C and further frozen for 16 hours at -20°C. After thawing, it was immersed to an enzyme solution by the procedure similar to Example 24, subjected to a reduced pressure treatment, enzyme reaction and enzyme deactivation. The content of oligosaccharides was measured from the obtained potato by the procedure similar to Example 24. The content of oligosaccharides was 6.77 g in 100 g of the potato. The results are shown in Figure 11 (steam cooked + freeze dried).

From the above results, the potato subjected to the dielectric heating treatment obtained in Example 24 maintained its shape and generated the largest amount of oligosaccharides inside the material. In comparison with the content of oligosaccharides included in an untreated raw potato, it increased approximately 6.6 times. Although the freezing treatment enabled an increase in the amount of oligosaccharides, the amount generated thereof by dielectric heating greatly exceeded it. It was confirmed that the dielectric heating treatment increased the efficiency of enzyme introduction into the cell most, and thus has the highest conversion efficiency of intracellular starch to oligosaccharides. Although the enzyme reaction with the ground product generated the largest amount of oligosaccharides, approximately 91% thereof could be converted to oligosaccharides in the inside of the food material while maintaining the shape by using the dielectric heating method. The shape, color and flavor of the potato were also equivalent to those of an ordinary potato, and the taste thereof had an increased sweetness due to an increase in the content of glucose.

### [Example 25]

A chicken white meat was adjusted into bars in 1.5 cm in height x 2.0 cm in width x 3.0 cm in length. A total weight of 25.7 g was heated in a microwave oven (NE-SV30HA: made by Matsushita Electric Industrial Co., Ltd.) at 500 W for 50 seconds. After it was cooled so that the product temperature reached 30°C, the water content of the chicken white meat was measured 69.2 wt%. It was measured 73.2 wt% before dielectric heating. After the chicken white meat was immersed to a degradative enzyme (Bromelain F: made by Amano Enzyme Inc.) having protease activity prepared at 0.5 wt% by using a phosphate buffer solution (pH 7.0) for 1 minute, it was taken out and subjected to a reduced pressure treatment (60 mmHg) by a vacuum pump for 5 minutes while adhering the enzyme solution on the surface thereof. It was returned to atmospheric pressure and subjected to a reaction for 1 hour in the refrigerator set at 4°C. After the reaction, enzyme deactivation was performed by heating for 5 minutes at 100°C. The treated chicken white meat maintained the shape prior to the treatment.

The obtained chicken white meat was ground and subjected to aqueous extraction, and the fraction of 10 kDa or less was prepared by ultrafiltration. The content of peptides included in the extracted fraction was measured by the Lowry method. The content of peptides was calculated as a BSA equivalent value. The content of peptides was 2.3 g in 100 g of the chicken white meat. The results are shown in Figure 12.

### [Comparative Example 25-1]

26.1 g of a chicken white meat was adjusted into bars by the procedure similar to Example 25, ground without further processing and subjected to aqueous extraction by the procedure similar to Example 25 to measure the content of peptides. The content of peptides was 0.24 g in 100 g of the chicken white meat. The results are shown in Figure 12.

### [Comparative Example 25-2]

25.5g of a chicken white meat was adjusted into bars by the procedure similar to Example 25 and immersed without being cooked to an enzyme solution prepared by the procedure similar to Example 25 for 1 hour at 4°C. Subsequently, it was taken out from the enzyme solution, and enzyme deactivation was performed by heating for 5 minutes at 100°C. It was then subjected to aqueous extraction by the procedure similar to Example 25 to measure the content of peptides. The content of peptides was 1.29 g in 100 g of the chicken white meat. The results are shown in Figure 12.

From the above results, when the enzyme was introduced after dielectric heating, the content of peptides increased most. The content of peptides increased approximately 10 times by performing enzyme contact and a pressure treatment after dielectric heating. Although the content of peptides increased when it was immersed to the enzyme for 1 hour, the amount of increase stayed approximately at 5 times. In the immersion method, excess enzyme reaction took place on the surface of the chicken white meat due to immersion in the enzyme solution for a long period of time, the shape could not be maintained due to collapse of the surface and the quality was impaired. A combined use of dielectric heating, enzyme contact and a pressure treatment was effective for increasing peptides while maintaining the shape. The content of amino acids as well as peptides also increased, and the taste was enhanced.

### [Example 26]

A burdock was cut into quasi-cylinders whose diameter was 2.0 cm and height was 1.0 cm. A total weight of 19.8 g was heated in a microwave oven (NE-SV30HA: made by Matsushita Electric Industrial Co., Ltd.) at 500 W for 70 seconds. After it was cooled so that the product temperature reached 30°C, the water content of the burdock was measured 78.6 wt%. It was measured 83.8 wt% before dielectric heating. After the burdock was immersed to a degradative enzyme (hemicellulase "Amano" 90: made by Amano Enzyme Inc.) prepared at 0.5 wt% by using a citrate buffer solution (pH 5.0) for 5 minutes, it was put in a pressurizer while immersing the burdock in the enzyme solution and subjected to an applied pressure state (700 atmospheric pressure) for 10 minutes. The burdock was returned to atmospheric pressure, taken out from the enzyme solution and subjected to a reaction for 1 hour in a constant temperature machine set at 70°C. After the reaction, enzyme deactivation was performed by braising for 5 minutes at 100°C. The obtained burdock maintained the shape prior to the treatment.

The manufactured burdock was ground and subjected to aqueous extraction. The content of dietary fibers included in the burdock was measured. The results are shown in Table 1. The content of dietary fibers in an untreated burdock was 3.9 wt%. The total amount of dietary fibers in the burdock after the enzyme reaction treatment remained almost unchanged at 4.0 wt%. However, the amount of insoluble dietary fibers included in a burdock was reduced to 2.6 wt%, while it was 3.0 wt% in the untreated burdock. The amount of water-soluble dietary fibers was increased to 1.4 wt% from 0.9 wt%.

The untreated burdock and burdock after the enzyme reaction treatment were ground, respectively, and the water-soluble fractions were extracted. When gastric retention times were examined in rat (Std: Wister/St, obtained from Shimizu Laboratory Suppliers Co., Ltd.), the extract from the burdock after the enzyme reaction treatment showed an approximately 2.5 times longer gastric retention time. Therefore, it was confirmed that the burdock obtained in the present invention is added with functionality while maintaining its color, flavor and shape.

A burdock was cut into quasi-cylinders whose diameter was 2.0 cm and height was 1.0 cm. A total weight of 19.8 g was heated in a microwave oven (National NE-SV30HA) at 500 W for 70 seconds. After it was cooled so that the product temperature reached 30°C, the water content of the burdock was measured 78.6 wt%. It was measured 83.8 wt% before dielectric heating. After the burdock was immersed to an enzyme solution (hemicellulase "Amano" 90: made by Amano Enzyme Inc.) having hemicellulase and pectinase activity prepared at 0.5 wt% by using a citrate buffer solution (pH 5.0) for 5 minutes, it was put in a pressurizer while immersing the burdock in the enzyme solution and subjected to an applied pressure state (700 atmospheric pressure) for 10 minutes. The burdock was returned to atmospheric pressure, taken out from the enzyme solution and subjected to a reaction for 1 hour in a constant temperature machine set at 70°C. After the reaction, enzyme deactivation was performed by braising for 5 minutes (100°C). The obtained burdock maintained the shape prior to the treatment.

The manufactured burdock was ground and subjected to aqueous extraction. The content of dietary fibers included in the burdock was measured. The results are shown in Table 1. The content of dietary fibers in an untreated burdock was 3.9 wt%. The total amount of dietary fibers in the burdock after the enzyme reaction treatment remained almost unchanged at 4.0 wt%. However, the amount of insoluble dietary fibers included in a burdock was reduced to 2.6 wt%, while it was 3.0 wt% in the untreated burdock. The amount of water-soluble dietary fibers was increased to 1.4 wt% from 0.9 wt%.

The untreated burdock and burdock after the enzyme reaction treatment were ground, respectively, and the water-soluble fractions were extracted. When gastric retention times were examined in rat (Std: Wister/St, obtained from Shimizu Laboratory Suppliers Co., Ltd.), the extract from the burdock after the enzyme reaction treatment showed an approximately 2.5 times longer gastric retention time. Therefore, it was confirmed that the burdock obtained in the present invention is added with functionality while maintaining its color, flavor and shape.

**Table 1**

| | Total amount of dietary fibers | Water-soluble | Insoluble |
|---|---|---|---|
| Prior to treatment | 3.9% | 0.9% | 3.0% |
| After treatment | 4.0% | 1.4% | 2.6% |

### Industrial Applicability

The food and process for producing a food of the present invention serve many uses as a food which retains the original shape and texture of the food material in an aging society, eases mastication and suppresses aspiration for those having difficulty in mastication and swallowing. Furthermore, it is extremely useful as an examination diet for medical use, wherein the diet can be used for examinations of mastication conditions, swallowing conditions, gastrointestinal tract activities, food movement speed and the like in those having difficulty in mastication and swallowing.

Moreover, it suppresses the outflow of components included in the food material, discoloration and disappearance of flavor, can hygienically produce a food without handling the soft food by directly touching by hand, is easy to handle, can be efficiently produced and serves extremely many uses. In particular, it can introduce a degradative enzyme and the like not only between the tissues of the food material but also inside the cell, can produce a further softened food and serves extremely many uses in an aging society.

## Claims

1. A food **characterized in that** the food retains the shape of the food material and uniformly comprises either one or both of a thickener and microorganism which generates a viscous material inside.

2. The food according to Claim 1, **characterized in that** the food uniformly comprises a degradative enzyme inside.

3. The food according to Claim 1 or 2, **characterized in that** the food uniformly comprises a contrast agent for medical use inside and is an examination diet for medical use.

4. A process for producing a food, **characterized in that** the process comprises bringing either one or both of a thickener in a non-solvated state and microorganism which generates a viscous material into contact with the surface of the food material; and performing a pressure treatment, such that the food retains the shape of the food material and uniformly includes either one or both of a thickener in a non-solvated state and microorganism which generates a viscous material inside.

5. The process for producing a food according to Claim 4, **characterized in that** either one or both of a thickener in a non-solvated state and microorganism which generates a viscous material, together with any one or more selected from an degradative enzyme, nutritious substance and seasoning, are brought into contact with the surface of the food material.

6. The process for producing a food according to Claim 4 or 5, **characterized in that** the method further comprises, after the pressure treatment, performing either one or both of solvation of the thickener in a non-solvated state and fermentation of the microorganism which generates a viscous material.

7. A process for producing a food, **characterized in that** the process comprises putting a food material brought into contact with a degradative enzyme and any one or more of a thickener in a non-solvated state, microorganism which generates a viscous material, nutritious substance and seasoning according to need, or putting a food material as well as a degradative enzyme, and any one or more of a thickener in a non-solvated state, microorganism which generates a viscous material, nutritious substance and seasoning according to need, in a packaging material for vacuum packaging such that the food retains the shape of the food material and includes the degradative enzyme inside uniformly; and cooking after softening the food material by the action of the degradative enzyme.

8. The process for producing a food according to any one of Claims 4 through 7, **characterized in that** the food material is used after dielectric heating, freezing, freezing-thawing or drying, or after dielectric heating followed by cooling to 60°C or less, then performing either one of freezing, freezing-thawing or drying.

9. A process for producing a food, **characterized in that** the process comprises bringing a degradative enzyme into contact with the surface of a food material after dielectric heating, freezing, freezing-thawing or drying of the food material, or after dielectric heating followed by cooling to 60°C or less, then performing either one of freezing, freezing-thawing or drying of the food material; and performing a pressure treatment, such that the food retains the shape of the food material and uniformly includes the degradative enzyme inside.

10. The process for producing a food according to any one of Claims 4 through 9, **characterized in that** the process comprises bringing a contrast agent for medical use into contact with a food material, or putting a contrast agent for medical use as well as a food material in a packaging material to manufacture an examination diet for medical use.
